# EUROPEAN PATENT APPLICATION

(11) **EP 4 752 230 A1**
(43) Date of publication of application: **03.06.2026**
(21) Application number: 24215884.8
(22) Date of filing: 27.11.2024
(51) Int. Cl.: C12Q 1/6806, C12Q 1/6844

(54) **METHODS FOR PRODUCING DNA PRODUCT**

(71) Applicant: 4basebio UK Ltd, Cambridge CB24 5QE (GB)
(72) Inventor: LANCKRIET, Heikki, Cambridge, CB24 5QE (GB); WALKER, Amy, Cambridge, CB24 5QE (GB); PAVLICKOVA, Milena, Cambridge, CB24 5QE (GB); BRUCE, Caitlyn, Cambridge, CB24 5QE (GB)
(74) Representative: Boult Wade Tennant LLP

(57) **Abstract**

The invention relates to methods for producing a linear deoxyribonucleic acid (DNA) product with enhanced resistance to nuclease digestion. The linear DNA product may be a closed linear DNA product, a linear DNA product comprising nuclease-resistant nucleotides, or a partially closed DNA product.

## Description

### TECHNICAL FIELD

The invention relates to methods for producing a linear deoxyribonucleic acid (DNA) product with enhanced resistance to nuclease digestion. The linear DNA product may be a closed linear DNA product, a linear DNA product comprising nuclease-resistant nucleotides, or a partially closed DNA product.

### BACKGROUND

Conventionally, synthetic DNA is produced using rolling circle amplification (RCA) from circular DNA templates, such as plasmids. Preparation of circular templates is often time consuming and costly. In addition, circular templates may contain unwanted bacterial antibiotic resistance genes or be associated with difficulties in the propagation of ITR sequences in *E.coli.* In order to generate a customized template DNA without bacterial sequences, DNA fragments may be cloned into a plasmid vector. The sequence of interest may sit between IoxP sites. The plasmid is then digested before being treated with Cre recombinase to re-circularize the sequence of interest, and remove any bacterial sequences sitting outside the IoxP sites. This generates a circular template compatible with an RCA process that is devoid of any exogenous sequence such as ori, antibiotic resistance gene, etc.

However, the current approaches to the production of synthetic DNA are not entirely synthetic as they involve bacterial propagation steps for the initial steps of template generation. Thus, a need exists for an entirely bacterial-free process for the generation of DNA templates suitable for RCA.

Moreover, prior to RCA, the DNA template must be denatured in order to allow binding of primase/polymerase enzymes (e.g. TthPrimPol) and/or primers to initiate the amplification reaction. This step is time-consuming and affects the stability of the DNA template. Specifically, the denaturation step may lead to the generation of large amounts of single-stranded DNA strands that can fold back upon themselves, forming intramolecular structures that are not favourable for the DNA replication process. Undesirable interactions can also occur intermolecularly between regions of partial complementarity. In addition, alkaline or heat-based denaturation might result in uneven denaturation across the DNA template. This can create unknown biases in the DNA template priming and replication fork formation. Thus, a need exists for a more efficient process for the production of synthetic DNA products.

### DESCRIPTION

The invention provides a method for producing a linear deoxyribonucleic acid (DNA) product with enhanced resistance to nuclease digestion. The present invention enables an entirely bacterial-free process for the generation of closed linear DNA templates suitable for RCA. The methods of the present invention do not require a circularized DNA template (e.g. a plasmid, a DNA circular template obtained from a Cre recombinase reaction, a cosmid, or a bacterial artificial chromosome (BACs)) for rolling circle amplification. The use of plasmids (or cosmids, or BACs) in the manufacture of a DNA product is associated with several drawbacks, e.g., the production of the plasmids (or cosmids, or BACs) is time-consuming and costly, there is a difficulty in the propagation of ITR sequences in *E.coli;* and the final DNA product may comprise antibiotic resistance markers originating from the plasmid. Similarly, the use of a DNA circular template obtained from a Cre recombinase reaction limits the type of DNA molecules that can be used and is time-consuming. Thus, the present invention allows for the generation of a fully synthetic linear DNA product from chemically manufactured gene fragments, making each step of the process synthetic.

Moreover, the methods of the present invention do not require a denaturation step during amplification, which has a positive effect on the stability of the template molecules. The present invention allows for efficient production of a linear DNA product with enhanced resistance to nuclease digestion. The methods of the invention may be performed in a single-pot, and without purification of different products throughout the different steps of the methods (optionally, the purification step may take place at the end of the method to produce a purer final product), which significantly reduces the time required for the production of the linear DNA product. The methods of the invention are therefore suitable for large-scale linear DNA product production for different applications in the biotechnology and pharmaceutical fields, including vaccine production, genetic engineering, gene therapy and personalized medicine.

In addition, by appending hairpin adaptors to linear dsDNA template molecules, the methods of the present invention:
- Generate closed linear DNA template molecules free of bacterial fermentation steps;
- Abolish the need for a denaturation step during amplification, because the primase/polymerase (e.g. TthPrimPol) and/or primer can bind within the non-complementary loop region (i.e. single stranded region) of the hairpin adaptor;
- Create minimal priming sites within the closed linear DNA template molecules, enhancing the quality of the amplified material;
- Maintain complex DNA sequences, e.g homopolymeric tracts such as polyA tails, or ITRs, that are prone to recombination in bacterial fermentation;
- Create a template that can be stored as a master template and re-amplified, as needed, mitigating the need for a master cell bank (MCB); and
- Reduce the chances of codirectional DNA polymerase collisions and therefore reduce the formation of abortive products. This is because the linear DNA product may comprise specific loop regions flanking the dsDNA cassette, which means that only two (distant) priming regions are available in the initial template.

The methods of the invention also allow for easy customization of the linear DNA product for downstream applications. For example, a desired sequence of overhangs can be added to the linear DNA product. Additionally, or alternatively, a functional portion may be added to the linear DNA product. Thus, overall, the present invention shows an improvement over the linear DNA production methods known in the art in terms of efficiency and flexibility with the sequences of the DNA molecules that can be used as templates and generated as products. The methods of the invention produce a fully synthetic DNA product.

The invention provides a method for producing a linear deoxyribonucleic acid (DNA) product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product of improved resistance to nuclease digestion.

The nuclease digestion may be exonuclease digestion (e.g. exonuclease I and/or exonuclease III digestion).

The linear DNA product may be a closed linear DNA product. Thus, the method for producing a closed DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the third adaptor molecule and closed at a second end by the fourth adaptor molecule.

The linear DNA product may be a linear DNA product comprising nuclease-resistant nucleotides. Thus, the method for producing a linear DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product, wherein the linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third and fourth adaptor molecules are nucleic acid molecules that comprise one or more nuclease-resistant nucleotides.

The linear DNA product may be a partially closed DNA product. The partially closed DNA product may be a partially closed linear DNA product. Thus, the method for producing a partially closed DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third adaptor molecule is a nucleic acid molecule that comprise one or more nuclease-resistant nucleotides, and wherein the linear double-stranded region is closed at the second end by the fourth adaptor molecule.

The invention provides a method for producing a linear deoxyribonucleic acid (DNA) product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification in the presence of at least one type of a nuclease-resistant nucleotide; and
(d) contacting the double-stranded DNA molecule with at least one endonuclease to generate the linear double-stranded DNA product, wherein the linear double-stranded DNA product has increased resistance to nucleases, such as exonucleases.

The step of contacting the linear dsDNA template molecule with a first hairpin adaptor and a second hairpin adaptor may further comprise contacting the linear dsDNA template molecule with an endonuclease. Thus, the reaction pot (e.g. a single reaction pot) may comprise the linear dsDNA template molecule, the first hairpin adaptor, the second hairpin adaptor and the endonuclease. The step of contacting the linear dsDNA template molecule with a first hairpin adaptor and a second hairpin adaptor may further comprise contacting the linear dsDNA template molecule with a ligase (e.g. T4 ligase or T7 ligase). Thus, the reaction pot (e.g. a single reaction pot) may comprise the linear dsDNA template molecule, the first hairpin adaptor, the second hairpin adaptor, the endonuclease, and the ligase. The endonuclease may be a Type IIS restriction endonuclease (e.g. Bsal).

The first hairpin adaptor may comprise a non-complementary loop region (i.e. a single stranded loop) of at least 10, at least 11, at least 12, least 13, at least 14, at least 15, least 16, at least 17, at least 18, least 19, at least 20, at least 21, least 22, at least 23, at least 24, least 25, at least 26, at least 27, least 28, at least 29, at least 30 nucleotides. Preferably, the first hairpin adaptor comprises a non-complementary loop region (i.e. a single stranded loop) of at least 14, or at least 29 nucleotides. The second hairpin adaptor may comprise a non-complementary loop region (i.e. a single stranded loop) of at least 10, at least 11, at least 12, least 13, at least 14, at least 15, least 16, at least 17, at least 18, least 19, at least 20, at least 21, least 22, at least 23, at least 24, least 25, at least 26, at least 27, least 28, at least 29, at least 30 nucleotides. Preferably, the second hairpin adaptor comprises a non-complementary loop region (i.e. a single stranded loop) of at least 14, or at least 29 nucleotides. The first hairpin adaptor and the second hairpin adaptor may be the same or different. Preferably, the first hairpin adaptor and the second hairpin adaptor are the same (i.e. have the same sequence, and the same non-complementary loop region).

The first hairpin adaptor and/or the second hairpin adaptor may comprise a primase/polymerase recognition sequence (i.e. a priming sequence). The primase/polymerase recognition sequence may be in the non-complementary loop region. The primase/polymerase recognition sequence may be XTC, where X stands for either of A, C, G, or T.

The first hairpin adaptor and/or the second hairpin adaptor may comprise an overhang.

The first hairpin adaptor and/or the second hairpin adaptor may comprise an endonuclease target sequence. Preferably, the endonuclease target sequence is present in the complementary portion of the first hairpin adaptor and/or the second hairpin adaptor. Thus, the at least one endonuclease target sequence in the closed linear DNA template molecule may originate from the first hairpin adaptor and/or the second hairpin adaptor.

The first hairpin adaptor and the second hairpin adaptor may have a longer non-complementary loop region that the third adaptor and the fourth adaptor molecules (in embodiments in which the third and the fourth adaptor molecules are also hairpins).

The linear dsDNA template molecule may comprise at least one endonuclease target sequence. Preferably, the endonuclease target sequence of the linear dsDNA template molecule is different to the endonuclease target sequence of the closed linear DNA template molecule. The endonuclease target sequence of the linear dsDNA template molecule allows for the appending (e.g. ligation) of the first hairpin adaptor and the second hairpin adaptor to the linear dsDNA template molecule. The endonuclease target sequence of the closed linear DNA template molecule allows for the appending (e.g. ligation) of the third and forth adaptor molecules.

Thus, the invention provides a method for producing a linear DNA product of improved resistance to nuclease digestion, wherein the method comprises:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the linear DNA product of improved resistance to nuclease digestion.

The method for producing a closed DNA product may comprise:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the third adaptor molecule and closed at a second end by the fourth adaptor molecule.

The method for producing a linear DNA product may comprise:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the linear DNA product, wherein the linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third and fourth adaptor molecules are nucleic acid molecules that comprise one or more nuclease-resistant nucleotides.

The method for producing a partially closed DNA product may comprise:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third adaptor molecule is a nucleic acid molecule that comprise one or more nuclease-resistant nucleotides, and wherein the linear double-stranded region is closed at the second end by the fourth adaptor molecule.

The invention provides a method for producing a linear DNA product of improved resistance to nuclease digestion, wherein the method comprises:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification in the presence of at least one type of a nuclease-resistant nucleotide; and
(d) contacting the double-stranded DNA molecule with at least one endonuclease to generate the linear double-stranded DNA product, wherein the at least one endonuclease of step (d) is different to the at least one endonuclease of step (b), and wherein the linear double-stranded DNA product has increased resistance to nucleases, such as exonucleases.

The method may further comprise a step of purification of the linear DNA product.

The method may further comprise a step of re-amplification of the linear DNA product. The step of re-amplification may be performed after the linear DNA product has been generated.

Preferably, steps (a)-(e) (or (a)-(d) if step (e) is not present) are performed in a single contiguous aqueous volume. That is to say that no purification needs to take place before the linear DNA product is produced. In some embodiments, a step of purification may take place during or after the preparation of a linear dsDNA template molecule. In some embodiments, a step of purification may take place after step (b), i.e. once the closed linear DNA template molecule has been generated.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a temperature of "about 22 °C" can include temperatures between 20 °C and 24°C.

Different sub-steps of the methods of the invention (as described above) are described in more detail in the sections below. It is to be understood that reaction conditions and reagents used in the methods as described below can be combined with different embodiments of the methods described herein. Section 1 describes method steps and reagents used up to (and including) the step of the generation of the closed linear DNA template molecule. Section 2 describes exemplary conditions for step (c), i.e. amplification of the closed linear DNA template molecule. Section 3 described experimental conditions for the generation of different linear DNA products (each having improved resistance to nuclease digestion). Section 4 describes further optional method steps. Section 5 describes uses and applications of the linear DNA products and Section 6 describes kits which comprise reagents that may be used in the methods of the invention. It is to be understood that the sections 1-4 below describe sub-sections of the methods of the invention which can be combined with each other. For example, the disclosure of Section 1 can be combined with Sections 2-4, etc. The disclosures of Sections 1-4 can also be combined with Sections 5 and/or 6.

### 1. Producing a closed linear DNA template molecule

The invention provides a method for producing a linear deoxyribonucleic acid (DNA) product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product of improved resistance to nuclease digestion.

The linear dsDNA template molecule may comprise at least one cleavable target sequence. The cleavable target sequence may be an endonuclease target sequence. Thus, the linear dsDNA template molecule may comprise at least one endonuclease target sequence. Preferably, the endonuclease target sequence of the linear dsDNA template molecule is different to the endonuclease target sequence of the closed linear DNA template molecule. The endonuclease target sequence of the linear dsDNA template molecule allows for the ligation of the first hairpin adaptor and the second hairpin adaptor to the linear dsDNA template molecule. Preferably, the linear dsDNA template molecule comprises at least two endonuclease target sequences. The endonuclease target sequences in the linear dsDNA template molecule may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQl, BtgZl, Esp3l,Sapl, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the linear dsDNA template molecule prior to the production of the linear DNA product.

Thus, the invention provides a method for producing a linear DNA product of improved resistance to nuclease digestion, wherein the method comprises:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the linear DNA product of improved resistance to nuclease digestion.

The method for producing a closed DNA product may comprise:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the third adaptor molecule and closed at a second end by the fourth adaptor molecule.

The method for producing a linear DNA product may comprise:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the linear DNA product, wherein the linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third and fourth adaptor molecules are nucleic acid molecules that comprise one or more nuclease-resistant nucleotides.

The method for producing a partially closed DNA product may comprise:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume, wherein the at least one endonuclease of step (d) is different to the at least one endonuclease of step (b); and
(e) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third adaptor molecule is a nucleic acid molecule that comprise one or more nuclease-resistant nucleotides, and wherein the linear double-stranded region is closed at the second end by the fourth adaptor molecule.

The invention provides a method for producing a linear DNA product of improved resistance to nuclease digestion, wherein the method comprises:
(a) providing a linear double stranded (ds)DNA template molecule, wherein the linear dsDNA template molecule comprises at least one endonuclease target sequence;
(b) contacting the linear dsDNA template molecule with a first hairpin adaptor, a second hairpin adaptor, a ligase and at least one endonuclease to generate a closed linear DNA template molecule, wherein the closed linear DNA template molecule comprises at least one endonuclease target sequence, wherein the at least one endonuclease target sequence of the closed linear DNA template molecule is different to the at least one endonuclease target sequence of the linear dsDNA template molecule;
(c) amplifying the closed linear DNA template molecule to generate a double-stranded DNA molecule, wherein the closed linear DNA template molecule is amplified by rolling circle amplification in the presence of at least one type of a nuclease-resistant nucleotide; and
(d) contacting the double-stranded DNA molecule with at least one endonuclease to generate the linear double-stranded DNA product, wherein the at least one endonuclease of step (d) is different to the at least one endonuclease of step (b), and wherein the linear double-stranded DNA product has increased resistance to nucleases, such as exonucleases.

For example, the at least one endonuclease from step (b) may be Bsal and the at least one endonuclease from step (d) may be Esp3I and/or BsmBl.

The method may further comprise a step of purification of the linear DNA product.

Preferably, the linear dsDNA template molecule is not circularized prior to amplification. Instead, the linear dsDNA template molecule is contacted with a first hairpin adaptor and a second hairpin adaptor to generate a closed linear DNA template molecule.

Preferably, the linear dsDNA template molecule does not comprise bacterial antibiotic genes. Preferably, the linear dsDNA template molecule does not originate from a plasmid, a cre-created template, a cosmid or a BAC. Preferably, the linear dsDNA template molecule does not originate from a circular template.

The step of contacting the linear dsDNA template molecule with a first hairpin adaptor and a second hairpin adaptor may further comprise contacting the linear dsDNA template molecule with an endonuclease. Thus, the reaction pot (e.g. a single reaction pot) may comprise the linear dsDNA template molecule, the first hairpin adaptor, the second hairpin adaptor and the endonuclease. The step of contacting the linear dsDNA template molecule with a first hairpin adaptor and a second hairpin adaptor may further comprise contacting the linear dsDNA template molecule with a ligase (e.g. T4 ligase or T7 ligase). Thus, the reaction pot (e.g. a single reaction pot) may comprise the linear dsDNA template molecule, the first hairpin adaptor, the second hairpin adaptor, the endonuclease, and the ligase. Thus, the step of contacting the linear dsDNA template molecule with a first hairpin adaptor and a second hairpin adaptor, and, optionally with an endonuclease and/or a ligase may be performed in a single contiguous aqueous volume. The step of contacting the linear dsDNA template molecule with a first hairpin adaptor and a second hairpin adaptor (and, optionally, an endonuclease and/or a ligase) may further comprise contacting the linear dsDNA template molecule with a nucleoside triphosphate. The nucleoside triphosphate may be ATP.

The endonuclease may be a Type IIS restriction endonuclease (e.g. Bsal). The endonuclease may be Bbsl, Bsal, BsmBI, BspQl, BtgZl, Esp3l,Sapl, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp11091, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI.

The ligase may be a T4 ligase and/or a T7 ligase.

The term "hairpin adaptor" as used herein is intended to encompass a nucleic acid adaptor molecule comprising a non-complementary loop region (i.e. a single stranded loop region) and a complementary region (i.e. a double stranded region). The hairpin adaptor may comprise an overhang or a blunt end. As it would be understood by a person skilled in the art, a single stranded oligonucleotide molecule may self-hybridize under specific experimental conditions to form a hairpin adaptor. For example, a single stranded oligonucleotide molecule may self-hybridize by incubating the molecule at high temperature (e.g. 80°C) for a period of time (e.g. 10 minutes), followed by gradual cooling to room temperature (about 22 °C) over a period of time (e.g. overnight). Thus, the methods described herein may comprise a step of self-hybridization of single stranded oligonucleotide molecules to form hairpin adaptors prior to the step of contacting the linear dsDNA template molecule with a first hairpin adaptor and a second hairpin adaptor.

The first hairpin adaptor may comprise a non-complementary loop region (i.e. a single stranded loop) of at least 10, at least 11, at least 12, least 13, at least 14, at least 15, least 16, at least 17, at least 18, least 19, at least 20, at least 21, least 22, at least 23, at least 24, least 25, at least 26, at least 27, least 28, at least 29, at least 30 nucleotides. Preferably, the first hairpin adaptor comprises a non-complementary loop region (i.e. a single stranded loop) of at least 15, or at least 29 nucleotides. The second hairpin adaptor may comprise a non-complementary loop region (i.e. a single stranded loop) of at least 10, at least 11, at least 12, least 13, at least 14, at least 15, least 16, at least 17, at least 18, least 19, at least 20, at least 21, least 22, at least 23, at least 24, least 25, at least 26, at least 27, least 28, at least 29, at least 30 nucleotides. Preferably, the second hairpin adaptor comprises a non-complementary loop region (i.e. a single stranded loop) of at least 15, or at least 29 nucleotides. The first hairpin adaptor and the second hairpin adaptor may be the same or different. Preferably, the first hairpin adaptor and the second hairpin adaptor are the same (i.e. have the same sequence, and the same non-complementary loop region).

The first hairpin adaptor and/or the second hairpin adaptor may comprise a primase/polymerase recognition sequence (i.e. a priming sequence). The primase/polymerase recognition sequence may be in the non-complementary loop region. The primase/polymerase recognition sequence may be XTC, where X stands for either of A, C, G, or T. The primase/polymerase recognition sequence may be recognized (i.e. primed) by a primase/polymerase, such as *Thermus thermophilus (Tth)* PrimPol (TthPrimPol).

As used herein, the term "priming" refers to the generation of an oligonucleotide primer on a polynucleotide template.

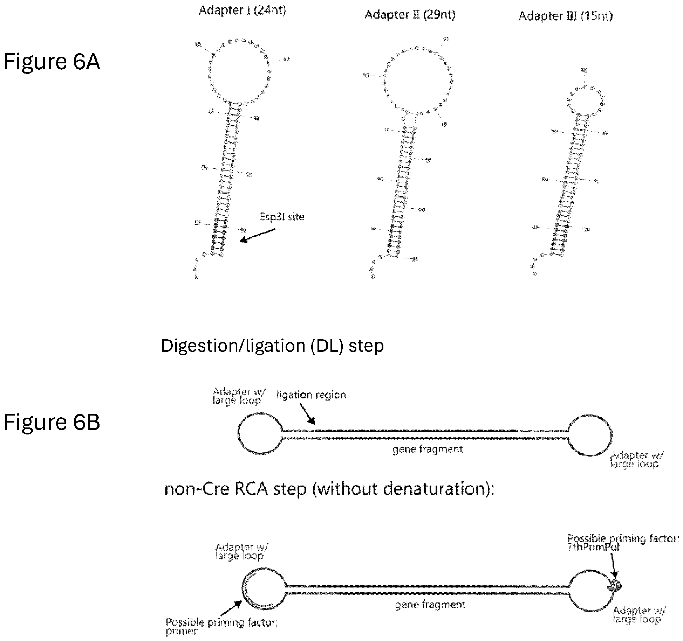

The first hairpin adaptor and/or the second hairpin adaptor may be any of the molecules shown in Fig. 6A. In the non-self-hybridized form, the first hairpin adaptor and/or the second hairpin adaptor may have a sequence of SEQ ID NO: 1, SEQ ID NO: 2, SEQ ID NO: 3 or SEQ ID NO: 10, or a sequence with at least 1, at least 2, at least 3, at least 4, or at least 5 nucleotide substitutions in SEQ ID NO: 1, SEQ ID NO: 2, SEQ ID NO: 3, or SEQ ID NO: 10. The non-complementary loop region of the first hairpin adaptor and/or the second hairpin adaptor may have a sequence of SEQ ID NO: 4, SEQ ID NO: 5, or SEQ ID NO: 6, or a sequence with at least 1, at least 2, at least 3, at least 4, or at least 5 nucleotide substitutions in SEQ ID NO: 4, SEQ ID NO: 5, or SEQ ID NO: 6.

The first hairpin adaptor and/or the second hairpin adaptor may comprise an overhang.

The first hairpin adaptor and/or the second hairpin adaptor may comprise an endonuclease target sequence. Preferably, the endonuclease target sequence is present in the complementary portion of the first hairpin adaptor and/or the second hairpin adaptor. Thus, the at least one endonuclease target sequence in the closed linear DNA template molecule may originate from the first hairpin adaptor and/or the second hairpin adaptor.

The first adaptor molecule and/or the second adaptor molecule may comprise a Type IIS endonuclease target sequence. The first adaptor molecule and/or the second adaptor molecule may comprise Bbsl, Bsal, BsmBI, BspQl, BtgZl, Esp3l,Sapl, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNI, Taqll, TspDTI and/or TspGWI target sequences. The first adaptor molecule and/or the second adaptor molecule may comprise a Cas nuclease-recognition sequence. For example, the first adaptor molecule and/or the second adaptor molecule may comprise a Cas9 nuclease-recognition sequence. Thus, the first adaptor molecule and/or the second adaptor molecule may comprise a protospacer adjacent motif (PAM) sequence.

The first adaptor molecule and/or the second adaptor molecule may comprise one or more locked nucleic acids (LNAs).

The first adaptor molecule and/or the second adaptor molecule may comprise one or more nuclease-resistant nucleotides (i.e. protected nucleotides), such as phosphorothioated nucleotides. The nuclease-resistant nucleotides may be located in the non-complementary loop region and/or the complementary region of the adaptor molecules. The nuclease-resistant nucleotides may be located in the overhang portion of the adaptor molecules.

The first adaptor molecule and/or the second adaptor molecule may confer resistance to 3'-end exonuclease digestion (e.g. by exonuclease III) and/or 5'-end exonuclease digestion (e.g. by exonuclease VIII).

The linear dsDNA template molecule may comprise a DNA cassette or a portion of the DNA cassette. The DNA cassette may comprise a gene of interest. The DNA cassette may comprise a promoter.

The linear dsDNA template molecule may be at least 1, at least 2, at least 3, at least 4, at least 5, at least 6, at least 7, at least 8, at least 9, at least 10, at least 11, at least 12, at least 13, at least 14, at least 15, at least 20, or at least 30 thousands nucleotides in length.

Optionally, the step of contacting the linear dsDNA template molecule with a first hairpin adaptor and a second hairpin adaptor to generate a closed linear DNA template molecule may be followed by a step of exonuclease digestion. The step of exonuclease digestion may be performed in the presence of exonuclease III, exonuclease I, exonuclease VIII, Lambda exonuclease and/or T7 exonuclease. The step of exonuclease digestion may be performed in the presence of exonuclease III and exonuclease I. The step of exonuclease digestion may be performed in the presence of exonuclease III, exonuclease I, Lambda exonuclease and T7 exonuclease.

The step of contacting the linear dsDNA template molecule with a first hairpin adaptor, a second hairpin adaptor, a ligase and at least one endonuclease to generate a closed linear DNA template molecule may be performed at a temperature of between 10°C-40°C. The step of contacting the linear dsDNA template molecule with a first hairpin adaptor, a second hairpin adaptor, a ligase and at least one endonuclease to generate a closed linear DNA template molecule may be performed at a first temperature of between 30°C -40°C and a second temperature of between 10°C-20°C. The step of contacting the linear dsDNA template molecule with a first hairpin adaptor, a second hairpin adaptor, a ligase and at least one endonuclease to generate a closed linear DNA template molecule may be performed at a first temperature of between 30°C -40°C for between 30 seconds and 5 minutes and a second temperature of between 10°C-20°C for between 30 seconds and 5 minutes. The step of contacting the linear dsDNA template molecule with a first hairpin adaptor, a second hairpin adaptor, a ligase and at least one endonuclease to generate a closed linear DNA template molecule may be performed at a first temperature of between 30°C-40°C for about 1 minute and a second temperature of between 10°C-20°C for about 1 minute. The step of contacting the linear dsDNA template molecule with a first hairpin adaptor, a second hairpin adaptor, a ligase and at least one endonuclease to generate a closed linear DNA template molecule may be performed at a first temperature of about 37°C for about 1 minute and a second temperature of about 16°C for about 1 minute. The step of contacting the linear dsDNA template molecule with a first hairpin adaptor, a second hairpin adaptor, a ligase and at least one endonuclease to generate a closed linear DNA template molecule may be performed under conditions such that the temperature cycles between the first temperature and the second temperature. The temperature cycles may comprise at least 10, at least 20 or at least 30 cycles.

### 1. Amplification

The invention provides a method for producing a linear deoxyribonucleic acid (DNA) product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product of improved resistance to nuclease digestion.

The invention also provides a method for producing a linear deoxyribonucleic acid (DNA) product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification in the presence of at least one type of a nuclease-resistant nucleotide; and
(d) contacting the double-stranded DNA molecule with at least one endonuclease to generate the linear double-stranded DNA product, wherein the linear double-stranded DNA product has increased resistance to nucleases, such as exonucleases.

Amplification (i.e. the step(s) of amplifying) may be performed using: a primer specific for the closed linear DNA template molecule; a pair of primers specific for the DNA template molecule; or performed in the presence of a primase/polymerase (such as *Thermus thermophilus (Tth)* PrimPol (TthPrimPol)). Primers specific for the DNA template molecule include single-stranded nucleic acids that are complementary to a target sequence in the DNA template molecule. A first primer of a set of primers may be complementary to the beginning of a target sequence in the DNA template molecule and a second primer of the set of primers may be complementary to the end of the target sequence in the DNA template molecule. In the case of a double-stranded DNA template, a first primer may be complementary to a first strand and a second primer may be complementary to a second strand.

Preferably, amplification (e.g. a rolling circle amplification) is performed without a denaturation step. That is to say that, in the preferred embodiment, the step of amplification utilizes enzymatic priming (e.g. using the primase/polymerase enzyme, such as TthPrimPol), rather than denaturation of the closed linear dsDNA template molecule.

The amplification process may be an *in vitro* or *in vivo* amplification process. Preferably, the amplification process is an *in vitro* amplification process.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA molecule may be generated by the rolling circle amplification *in vitro* under isothermal conditions using a suitable nucleic acid polymerase, such as Phi29 DNA polymerase.

Rolling circle amplification may take place in the presence of the closed linear DNA template molecule and deoxynucleotide triphosphates (dNTPs). Rolling circle amplification may take place in the presence of the closed linear DNA template molecule, deoxynucleotide triphosphates (dNTPs), and DNA polymerase (e.g. a phi29 DNA polymerase or a chimeric form thereof (e.g. QualiPhi^{™} DNA polymerase). Rolling circle amplification may take place in the presence of the closed linear DNA template molecule, deoxynucleotide triphosphates (dNTPs), DNA polymerase (e.g. a phi29 DNA polymerase or a chimeric form thereof (e.g. QualiPhi^{™} DNA polymerase) and DNA primase (e.g. TthPrimPol). Rolling circle amplification may take place in the presence of the closed linear DNA template molecule, deoxynucleotide triphosphates (dNTPs), DNA polymerase (e.g. a phi29 DNA polymerase or a chimeric form thereof (e.g. QualiPhi^{™} DNA polymerase) and polymerase pyrophosphatase. Rolling circle amplification may take place in the presence of the closed linear DNA template molecule, deoxynucleotide triphosphates (dNTPs), DNA polymerase (e.g. a phi29 DNA polymerase or a chimeric form thereof (e.g. QualiPhi^{™} DNA polymerase), DNA primase (e.g. TthPrimPol) and polymerase pyrophosphatase. Additionally, rolling circle amplification may take place in the presence of at least one type of a nuclease-resistant nucleotide.

The rolling circle amplification reaction may be incubated for 10-30 hours at a temperature of between 25°C-35°C, followed a 1-60 minutes incubation at a temperature of between 50-60°C. For examples, the rolling circle amplification reaction may be incubated for 20 hours at a temperature of 30°C, followed a 10 minutes incubation at a temperature of 55°C.

As used herein the term "protected nucleotide" or "nuclease-resistant nucleotide" is intended to encompass any type of molecule that provides or enhances resistance to nuclease digestion (especially exonuclease digestion).

Amplification may take place in the presence of at least one type of a nuclease-resistant nucleotide. Amplification may take place in the presence of at least two types of a nuclease-resistant nucleotide. Amplification may take place in the presence of at least one type of phosphorothioated nucleotides.

The nucleotides resistant to exonuclease digestion (i.e. protected nucleotides) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate, preferably α-S-dATP, α-S-dGTP or α-S-dTTP.

Amplification may take place in the presence of at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

Amplification may take place in the presence of at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

Amplification may take place in the presence of at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

Preferably, the nuclease-resistant nucleotide is a phosphorothioated nucleotide.

The ratio of phosphorothioated nucleotides to total nucleotides used in the methods described herein may be at least 0.0001, at least 0.0025, at least 0.01, at least 0.025, at least 0.05, at least 0.075, at least 0.10, at least 0.12 at least 0.15, at least 0.25, at least 0.35, at least 0.5, or at least 0.75. The ratio of the phosphorothioated nucleotides to total nucleotides used in the methods described herein may be less than 1, less than 0.9, less than, 0.8, less than 0.65, less than 0.5, less than 0.4, less than 0.3, less than 0.2, less than 0.1, less than 0.075, or less than 0.05. The ratio of the phosphorothioated nucleotides to total nucleotides may be between 0.0001-1, between 0.0025-0.75, between 0.025-0.65, between 0.025-0.15, or between 0.25-0.50. Preferably, a ratio of the phosphorothioated nucleotides to total nucleotides is between 0.025-0.15.

### 2. Producing linear DNA products

The invention provides a method for producing a linear deoxyribonucleic acid (DNA) product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product of improved resistance to nuclease digestion.

The linear DNA product may be resistant to nuclease digestion or may have improved or enhanced resistance to nuclease digestion. The linear DNA product may be resistant to exonuclease digestion or have improved or enhanced resistance to exonuclease digestion. The linear DNA product may be resistant, or have improved or enhanced resistance, to digestion by exonucleases that cleave the 3'-end nucleotides (e.g. exonuclease III) and/or exonucleases that cleave the 5'-end nucleotides (e.g. exonuclease VIII). The terms "improved" or "enhanced" in the context of resistance to enzyme digestion refer to higher resistance to enzyme digestion when compared to a DNA product not produced by the methods described herein. For example, when compared to a product that does not comprise protected nucleotides, such as phosphorothioated nucleotides.

Preferably, the linear DNA product is a linear double-stranded DNA product.

The linear DNA product may be a closed linear DNA product. Thus, the method for producing a closed DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the third adaptor molecule and closed at a second end by the fourth adaptor molecule.

The linear DNA product may be a partially closed DNA product. The partially closed DNA product may be a partially closed linear DNA product. Thus, the method for producing a partially closed DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third adaptor molecule is a nucleic acid molecule that comprise one or more nuclease-resistant nucleotides, and wherein the linear double-stranded region is closed at the second end by the fourth adaptor molecule.

The linear DNA product may be a linear DNA product comprising nuclease-resistant nucleotides. Thus, the method for producing a linear DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product, wherein the linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third and fourth adaptor molecules are nucleic acid molecules that comprise one or more nuclease-resistant nucleotides.

The third adaptor molecule and/or the fourth adaptor molecule may comprise at least 1, at least 2, at least 3, at least 4, or at least 5 nuclease (e.g. exonuclease)- resistant nucleotides. The third adaptor molecule and/or the fourth adaptor molecule may comprise at least 1, at least 2, at least 3, at least 4, or at least 5 phosphorothioated nucleotides.

The third adaptor molecule may comprise or consist of the sequence of SEQ ID NO: 7, or a sequence comprising at least 1 or at least 2 nucleotide substitutions in the SEQ ID NO: 7. The fourth adaptor molecule may comprise or consist of the sequence of SEQ ID NO: 8 (e.g. top strand) and SEQ ID NO: 9 (e.g. bottom strand), or a sequence comprising at least 1 or at least 2 nucleotide substitutions in the SEQ ID NO: 8 and/or SEQ ID NO: 9.

In an embodiment, the third adaptor molecule may not comprise nuclease-resistant (e.g. phosphorothioated) nucleotides and the fourth adaptor molecule may comprise nuclease-resistant (e.g. phosphorothioated) nucleotides. In an embodiment, the third adaptor molecule may be a hairpin adaptor and the fourth adaptor molecule may comprise nuclease-resistant (e.g. phosphorothioated) nucleotides. The third adaptor molecule and/or the fourth adaptor molecule may comprise a 5' phosphate.

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to nuclease digestion (e.g. exonuclease digestion). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The linear DNA product may have enhanced resistance to nuclease digestion (e.g. exonuclease digestion). The enhanced resistance to nuclease (e.g. exonuclease) digestion may extend the life of the linear DNA product in a cell (i.e. the linear DNA product has enhanced resistance to intracellular exonucleases) and in a cell-free system (i.e. the linear DNA product has enhanced resistance to extracellular exonucleases). The enhanced resistance to exonuclease digestion may be provided by appending (e.g. ligating) a third adaptor molecule to a first end of the linear double-stranded region and a fourth adaptor to a second end of the linear double stranded region. In this regard, the entire content of WO2023/006978 A1 is incorporated herein by reference.

The third adaptor molecule and/or the fourth adaptor molecule may both be linear adaptor molecules comprising nuclease resistant nucleotides (i.e. forming a linear open-ended DNA product).

The third adaptor molecule and/or the fourth adaptor molecule may each comprise a hairpin or a stem-loop (i.e. forming a closed linear DNA product). Thus, the third adaptor molecule and/or the fourth adaptor molecule may comprise a non-complementary loop region and a double stranded complementary region.

The third adaptor molecule may be a linear adaptor molecule comprising nuclease resistant nucleotides and the fourth adaptor molecule may comprise a hairpin or a stem-loop (or vice versa), (i.e. forming a partially closed DNA product). The partially closed DNA product is typically a partially closed linear DNA product. The partially closed DNA product may be a partially covalently closed DNA product.

The adaptor molecules may provide protection from digestion by exonucleases that cleave the 3'-end nucleotides (e.g. exonuclease III) and exonucleases that cleave the 5'-end nucleotides (e.g. exonuclease VIII). Thus, the linear DNA product may be resistant to exonuclease digestion. Preferably, the linear DNA product is resistant to 3'- and 5'-exonuclease digestion.

The third and fourth adaptor molecules may be identical molecules, or they may be different molecules. For example, the third adaptor molecule and/or the fourth adaptor molecule may comprise a hairpin. The third adaptor molecule and/or the fourth adaptor molecule may be double-stranded linear nucleic acid molecules comprising one or more nuclease-resistant nucleotides. The third adaptor molecule may comprise a hairpin and the fourth adaptor molecule may be a double-stranded linear nucleic acid molecule comprising one or more nuclease-resistant nucleotides. The linear DNA product produced by the methods described herein may be resistant to nuclease (e.g. exonuclease) digestion.

The third adaptor molecule may be a nucleic acid adaptor molecule (e.g. DNA). The fourth adaptor molecule may be a nucleic acid adaptor molecule (e.g. DNA). The third adaptor molecule and/or the fourth adaptor molecule may be a synthetic adaptor molecule. The third adaptor molecule and/or the fourth adaptor molecule may not be a plasmid or a vector DNA.

The third adaptor molecule and/or the fourth adaptor molecule may comprise at least 1, at least 2, at least 3, at least 4, or at least 5 modified nucleotide(s) (e.g. alkynyl modified nucleotide(s) or an amino group (i.e. NH₂) to enable the appending of a functional portion (e.g. nuclear localization signal (NLS) molecule).

The third adaptor molecule and/or the fourth adaptor molecule may comprise an overhang. For example, the third adaptor molecule and/or the fourth adaptor molecule may comprise a 5' overhang or a 3' overhang. The third adaptor molecule and/or the fourth adaptor molecule may comprise a blunt end. The overhang may have at least 3 nucleotides (preferably from 4 to 6 nucleotides). The overhang of the third adaptor molecule and/or the fourth adaptor molecule may be complementary to the first and/or second end of the linear double-stranded region. The overhang of the third adaptor molecule and/or the fourth adaptor molecule may anneal to the first and/or second end of the linear double-stranded region.

The third adaptor molecule and/or the fourth adaptor molecule may comprise a self-complementary element which creates a loop, such as a hairpin loop or a stem- loop. That is to say that the adaptor may initially be provided in a linear form, and in the methods described herein may fold on itself to form a hairpin loop or a stem-loop. The third adaptor molecule may comprise a hairpin or a stem-loop. The fourth adaptor molecule may comprise a hairpin or a stem-loop. Both the third and fourth adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides.

Preferably the 3' overhang or the 5' overhang is 4-8 nucleotides. Each end of the linear double-stranded region may comprise a 3' or a 5' overhang.

The third adaptor molecule and/or the fourth adaptor molecule may comprise a single-stranded portion. The single-stranded portion may form a hairpin or a stem-loop. Thus, the third adaptor molecule and/or the fourth adaptor molecule may comprise a loop portion. The single-stranded portion may be at least 5, at least 10, at least 12, at least 14, at least 15, at least 16, at least 18, at least 20, at least 22, at least 24, at least 26, at least 28, at least 30, at least 32, at least 34, at least 36, at least 38, or at least 40 nucleotides. Preferably, the single-stranded portion is at least 10, at least 12 or at least 14 nucleotides.

The third adaptor molecule and/or the fourth adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs. Preferably, the double-stranded portion is at least 10 base pairs long.

The third adaptor molecule and/or the fourth adaptor molecule may comprise a 5' phosphate. The 5' phosphate may facilitate ligation to the linear double-stranded region (which may comprise a 3'-OH group at first and/or second ends). The third adaptor molecule and/or the fourth adaptor molecule may comprise a 3'-OH. The 3'-OH may facilitate ligation to the linear double-stranded region (which may comprise a 5' phosphate at first and/or second ends).

The third adaptor molecule may comprise a portion (e.g. the overhang) that is complementary to the first end of the linear double-stranded region. The fourth adaptor molecule may comprise a portion (e.g. the overhang) that is complementary to the second end of the linear double-stranded region. The third adaptor molecule may comprise a portion that anneals to the first end of the linear double-stranded region. The fourth adaptor molecule may comprise a portion that anneals to the second end of the linear double-stranded region. The third adaptor molecule may comprise a portion that is complementary and anneals to the first end of the linear double-stranded region. The fourth adaptor molecule may comprise a portion that is complementary and anneals to the second end of the linear double-stranded region.

The portion that is complementary or anneals to the first or second end of the linear double-stranded region may be a 5' overhang or a 3' overhang of the third and/or fourth adaptor molecule. The overhang of the third adaptor molecule may be complementary to the first end of the linear double-stranded region and/or the overhang of the fourth adaptor molecule may be complementary to the second end of the linear double-stranded region. The overhang of the third adaptor molecule may anneal to the first end of the linear double-stranded region and/or the overhang of the fourth adaptor molecule may anneal to the second end of the linear double-stranded region. The overhang of the third adaptor molecule may be complementary to and anneal to the first end of the linear double-stranded region and/or the overhang of the fourth adaptor molecule may be complementary to and anneal to the second end of the linear double-stranded region.

The third adaptor molecule and/or the fourth adaptor molecule may comprise a Type IIS endonuclease target sequence. The third adaptor molecule and/or the fourth adaptor molecule may comprise Bbsl, Bsal, BsmBI, BspQl, BtgZl, Esp3I,SapI, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequences.

The third adaptor molecule and/or the fourth adaptor molecule may comprise one or more locked nucleic acids (LNAs).

The third adaptor molecule and/or the fourth adaptor molecule may comprise one or more nuclease-resistant nucleotides (i.e. protected nucleotides), such as phosphorothioated nucleotides. The nuclease-resistant nucleotides may be located in the single-stranded portion (e.g. hairpin portion) or the double-stranded portion of the adaptor molecules. The nuclease-resistant nucleotides may be located in the overhang portion of the adaptor molecules.

The third adaptor molecule and/or the fourth adaptor molecule may confer resistance to 3'-end exonuclease digestion (e.g. by exonuclease III) and/or 5'-end exonuclease digestion (e.g. by exonuclease VIII).

The closing of the linear double-stranded region at the first end may generate a first closed end of the closed linear DNA product. The closing of the linear double-stranded region at the second end may generate a second closed end of the closed linear DNA product. The first closed end and the second closed end of the closed linear DNA product may be resistant to nuclease digestion. The nuclease digestion may be exonuclease digestion. Preferably, the first closed end and the second closed end of the closed linear DNA product confer resistance to 3'-end exonuclease digestion (e.g. by exonuclease III) and/or 5'-end exonuclease digestion (e.g. by exonuclease VIII).

The third adaptor molecule and/or the fourth adaptor molecule may comprise one or more nuclease-resistant nucleotides (e.g. phosphorothioated nucleotides), such that, once the adaptor molecules are appended (e.g. ligated) to the linear double-stranded region, the linear DNA product is resistant to nuclease digestion or has improved or enhanced resistance to nuclease digestion. The linear DNA product may be resistant to 3'-end exonuclease digestion (e.g. by exonuclease III) and/or 5'-end exonuclease digestion (e.g. by exonuclease VIII). The linear DNA product may be resistant to exonuclease III, exonuclease I, T7 exonuclease, and/or Lambda exonuclease digestion.

Each adaptor molecule may comprise a plurality of phosphorothioated nucleotides. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

Each adaptor molecule may be double-stranded (e.g. double-stranded DNA). Each adaptor molecule may comprise a portion that is double-stranded (e.g. double-stranded DNA).

The third adaptor molecule and/or the fourth adaptor molecule may each comprise at least 3, at least 4, at least 5, at least 6, at least 7, at least 8, at least 9, at least 10, at least 11, at least 12, at least 13, at least 14, at least 15, or at least 16 base pairs.

Each adaptor molecule may comprise a plurality of phosphorothioated nucleotides in each strand. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand. Preferably, the adaptor molecule comprises at least 5 phosphorothioated nucleotides. Preferably, the adaptor molecule comprises at least 5 phosphorothioated nucleotides in each strand.

Each adaptor molecule may comprise at least 5%, at least 10%, at least 20%, at least 30%, at least 40%, at least 50%, at least 60%, at least 70%, at least 80%, at least 90%, at least 95%, or at least 100% of nuclease-resistant nucleotides.

The nuclease-resistant nucleotides (i.e. nucleotides resistant to exonuclease digestion) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The adaptor molecules may comprise at least four types of phosphorothioated nucleotides. For example, the at least four types of nuclease-resistant nucleotides are α-S-dATP, α-S-dCTP, α-S-dGTP and α-S-dTTP.

The nuclease-resistant nucleotides (i.e. nucleotides resistant to exonuclease digestion) may be 2'-O-methyl nucleotides or 2'-O-methoxyethyl (MOE) nucleotides. The nuclease-resistant nucleotides may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The third adaptor molecule and/or the fourth adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, an RNA sequence or a DNA/RNA chimera sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enables the detection of the double-stranded nucleic acid molecule via, physical, chemical and/or biological means. The label may be a chromophore, a fluorophore and/or a radioactive molecule.

To facilitate detection and/or quantification of the DNA product, the functional portion may comprise a fluorophore, a radioactive compound or a barcode.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeqTM, MiSeqTM and/or Genome AnalyzerTM sequencing systems), Oxford NanoporeTM Technologies (e.g. the MinION sequencing system), Ion TorrentTM (e.g. the Ion PGMTM and/or Ion ProtonTM sequencing systems), Pacific Biosciences (e.g. the PACBIO RS II sequencing system); Life TechnologiesTM (e.g. a SOLiD sequencing system), Roche (e.g. the 454 GS FLX+ and/or GS Junior sequencing systems), or any other sequencing platform of interest.

The third adaptor molecule and/or the fourth adaptor molecule may comprise an inverted terminal repeat sequence. The inverted terminal repeat sequences of the third adaptor molecule and the fourth adaptor molecule may be symmetrical (i.e. have the same symmetrical three-dimensional organization with respect to each other) or asymmetrical (i.e. have different three-dimensional organization with respect to each other). The inverted terminal repeat sequences of the third adaptor molecule and the fourth adaptor molecule may be from the same or different serotypes. An inverted terminal repeat sequence may comprise a terminal resolution site and a Rep binding site.

The third adaptor molecule and/or the fourth adaptor molecule may comprise an aptamer.

The expression "at a first end" or "at a second end" of the linear double-stranded region as used herein in the context of the third adaptor molecule and/or the fourth adaptor molecule means that the adaptors are attached to the ends of the linear double-stranded region. The first end may be the 5' end of the linear double-stranded region. The second end may be the 3' end of the linear double-stranded region. The adaptors may be attached to the linear double-stranded region by annealing complementary nucleotides (e.g. if the adaptor has an overhang), and/or by ligation. The adaptors and/or the linear double-stranded region may be pre-processed by digesting (with an endonuclease) the restriction enzyme sites to allow annealing and/or ligation of the adaptors to the linear double-stranded region.

As discussed herein, different types of adaptor molecules may be appended to the linear double-stranded region. Depending on the type of adaptor molecules used, the linear DNA product of the present invention may be an open-ended linear DNA product, a partially closed linear DNA product or a closed linear DNA product.

The linear DNA product may be an open-ended linear DNA product, optionally wherein the linear DNA product comprises a third adaptor molecule at a first end and a fourth adaptor molecule at a second end. Preferably, the third and the fourth adaptor molecules each comprise at least one nuclease resistant nucleotide.

The linear DNA product may be a closed linear DNA product, optionally wherein the linear double-stranded region is closed at a first end by a third adaptor molecule and closed at a second end by a fourth adaptor molecule. Preferably, wherein the third and the fourth adaptor molecules are each hairpin molecules. The linear double-stranded region may be closed at a first end by protelomerase (e.g. TeIN) or closed at a second end by protelomerase (e.g. TeIN), optionally wherein a third adaptor molecule comprises a protelomerase target sequence or a portion thereof and/or a fourth adaptor molecule comprises a protelomerase target sequence or a portion thereof. The linear double-stranded region may be closed at a first end by a third adaptor molecule and closed at a fourth end by protelomerase (e.g. TeIN) (optionally wherein a second adaptor molecule comprises a protelomerase target sequence or a portion thereof). The linear double-stranded region may be closed at a first end by protelomerase (optionally wherein a third adaptor molecule comprises a protelomerase target sequence or a portion thereof) and closed at a second end by a fourth adaptor molecule.

The linear double-stranded region may be a partially closed linear DNA, optionally wherein the linear double-stranded region is closed at a first end by a third adaptor molecule or closed at a second end by a fourth adaptor molecule. Preferably, wherein the third adaptor molecule or the fourth adaptor molecule is a hairpin molecule. The partially closed linear double-stranded region may be closed at a first end by protelomerase (e.g. TeIN), optionally wherein a third adaptor molecule comprises a protelomerase target sequence or a portion thereof. The partially closed linear double-stranded region may be closed at a second end by protelomerase (e.g. TeIN), optionally wherein a fourth adaptor molecule comprises a protelomerase target sequence or a portion thereof.

The linear DNA product may comprise a DNA cassette. The linear double-stranded region of the linear DNA product may comprise a DNA cassette. In some embodiments, the DNA cassette may be difficult to completely and/or faithfully amplify from a single DNA template molecule. DNA cassettes may be difficult to completely and/or faithfully amplify from a single DNA template molecule due to their size and/or sequence composition (e.g. high GC content and/or presence of repeat regions such as palindromic repeats). In those embodiments, a part of the DNA cassette may be provided by the third adaptor molecule and/or the fourth adaptor molecule. The DNA cassette may be formed of at least 3, at least 4, at least 5, at least 6, at least 7, at least 8, at least 9 or at least 10 cassette sequences. The DNA cassette may be formed of n cassette sequences, wherein n is between 1-100, preferably, between 1-10.

The linear DNA product may comprise a single-stranded DNA cassette or a double-stranded DNA cassette.

Each of the cassette sequences may provide a different sequence of the DNA cassette. Alternatively, at least 2, at least 3, at least 4, at least 5, at least 6, at least 7, at least 8, at least 9 or at least 10 cassette sequences, may provide the same sequence of the DNA cassette. The DNA cassette may be formed of at least 2, at least 3, at least 4, at least 5, at least 6, at least 7, at least 8, at least 9, or at least 10 repeats of the same sequence.

The skilled person would be aware of a range of options for the composition of the DNA cassette and sequence components that might be included in such a cassette.

The DNA cassette may be at least 1, at least 2, at least 3, at least 4, at least 5, at least 6, at least 7, at least 8, at least 9, at least 10, at least 11, at least 12, at least 13, at least 14 or at least 15 kilobases (kb) in length. Thus, the linear double-stranded portion may be at least 1, at least 2, at least 3, at least 4, at least 5, at least 6, at least 7, at least 8, at least 9, at least 10, at least 11, at least 12, at least 13, at least 14, at least 15, at least 20, or at least 30 kilobases (kb) in length. In the embodiments in which the DNA cassette is single-stranded, the DNA cassette may be at least 1, at least 2, at least 3, at least 4, at least 5, at least 6, at least 7, at least 8, at least 9, at least 10, at least 11, at least 12, at least 13, at least 14 or at least 15 kilobases (kb) in length. Thus, the linear double-stranded portion may be at least 1, at least 2, at least 3, at least 4, at least 5, at least 6, at least 7, at least 8, at least 9, at least 10, at least 11, at least 12, at least 13, at least 14, at least 15, at least 20, or at least 30 thousands nucleotides in length.

The DNA cassette (or a cassette sequence) may comprise a GC content of at least 50%, at least 60%, at least 65%, at least 70%, at least 75%, at least 80%, at least 85%, at least 90%, at least 95%, at least 100%. Preferably, the DNA cassette (or a cassette sequence) may have a GC content of at least 60%. More preferably, the DNA cassette (or a cassette sequence) may have a GC content of at least 65%. GC content may refer to the percentage of bases that are either guanine or cytosine.

The DNA cassette (or a cassette sequence) may comprise a repeated sequence. Repeated sequence may refer to a sequence in which two or more identical or extremely similar nucleotide sequences are repeated. A repeated sequence includes but is not limited to a tandem repeat or interspersed repeat. The repeated sequence may be a direct repeat or inverted repeat (e.g. a palindromic repeat). Tandem repeats are repeated sequences which are directly adjacent to each other. Interspersed repeats are repeated sequences which are found in different locations. Direct repeats occur when a nucleotide sequence is repeated with the same directionality. Inverted repeats occur when a nucleotide sequence is repeated in the inverse direction. When there are no nucleotides separating the inverted repeat, the sequence is called a palindromic repeat. The repeated sequence may be at least 2, 5, 10, 25, 50, 75, 100, 250, 500 or 750 nucleotides long. Preferably, the repeated sequence is at least 100 nucleotides long. The repeated sequence may be 2-1000 nucleotides long, 3-500 nucleotides long, 4-250 nucleotides long, 5-100 nucleotides long, 10-1000 nucleotides long, 15-500 nucleotides long, 20-250 nucleotides long, 25-100 nucleotides long. Preferably, the repeated sequence is 4-250 nucleotides long.

The DNA cassette may comprise inverted terminal repeat sequences upstream and downstream of a coding region. The inverted terminal repeat sequences may be symmetrical (i.e. have the same symmetrical three-dimensional organization with respect to each other) or asymmetrical (i.e. have different three-dimensional organization with respect to each other). The inverted terminal repeat sequences may be from the same or different serotypes. An inverted terminal repeat sequence may comprise a terminal resolution site and a Rep binding site.

The DNA cassette (or a cassette sequence) may comprise a homopolymeric sequence. The DNA cassette (or a cassette sequence) may comprise a homopolymeric sequence at a 5'-end or a 3'-end or both a 5'-end and a 3'-end. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides. The homopolymeric sequence may be formed by at least two cassette sequences.

The DNA cassette (or a cassette sequence) may encode a nuclease protein of a CRISPR system (e.g. Cas9, Cpf1 or Mad7), and/or a guide RNA, a DNA polymerase, a reverse transcriptase, or an exonuclease. The DNA cassette (or a cassette sequence) may encode a DNA polymerase, a reverse transcriptase or an exonuclease. The DNA cassette (or a cassette sequence) may encode a nuclease protein of a CRISPR system (e.g. Cas9, Cpf1, or Mad7) and a DNA polymerase, a reverse transcriptase or an exonuclease.

The DNA cassette (or a cassette sequence) may encode a therapeutic target and a T7 or SP6 promoter. The therapeutic target may comprise or consist of functional gene, vaccine antigen or neoantigen. The therapeutic target may be at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 base pairs long.

The DNA cassette (or a cassette sequence) may encode any component of self-amplifying mRNA system. The linear DNA product may comprise (or further comprise) a gene sequence encoding for all the components of a self-amplifying mRNA system. The DNA cassette (or a cassette sequence) may encode tRNA or siRNA.

The DNA cassette (or a cassette sequence) may encode the nuclease of the CRISPR system and/or the guide RNA used to treat any disease or disorder. For example, the linear DNA product encoding the nuclease of the CRISPR system and the linear DNA product encoding the guide RNA may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product encoding the nuclease of the CRISPR system and the linear DNA product encoding the guide RNA is used to treat a monogenic disorder. For example, the linear DNA product encoding the nuclease of the CRISPR system and the linear DNA product encoding the guide RNA, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The DNA cassette (or a cassette sequence) may comprise a coding sequence. The coding sequence may be at least 250, at least 500, at least 750, at least 1000, at least 1500, at least 2000, at least 3000, at least 4000, at least 5000, at least 7500, at least 10,000, at least 15,000, at least 20,000. at least 25,000, or at least 30,000 base pairs (bp) in length. The coding sequence may be formed by at least two cassette sequences. In the embodiments, in which the DNA cassette is single-stranded, the coding sequence may be at least 250, at least 500, at least 750, at least 1000, at least 1500, at least 2000, at least 3000, at least 4000, at least 5000, at least 7500, at least 10,000, at least 15,000, at least 20,000. at least 25,000, or at least 30,000 nucleotides in length

The DNA cassette (or a cassette sequence) may comprise a promoter (or a portion thereof) and a coding sequence. The promoter (or portion thereof) may be operably linked to the coding sequence. The cassette may comprise a promoter, a coding sequence and a 3' untranslated region e.g. a poly(A) tail. The cassette may comprise a promoter, a coding sequence, a 3' untranslated region e.g. a poly(A) tail, and a translational termination sequence. The cassette may comprise a promoter, a ribosome binding site, a coding sequence and a 3' untranslated region e.g. a poly(A) tail. The cassette may comprise a promoter, a ribosome binding site, a coding sequence, a 3' untranslated region e.g. a poly(A) tail, and a translational termination sequence. The cassette may comprise one or more of: a promoter; a ribosome binding site; a coding sequence; a 3' untranslated region e.g. a poly(A) tail; and a translational termination sequence.

The DNA cassette may comprise a promoter. The promoter may be an inducible or constitutive promoter. The promoter may be, for example, a CMV promoter or a CAG promoter.

The DNA cassette may further comprise an enhancer.

The DNA cassette may comprise an insulator. The insulator may be a cis-regulatory element. The insulator may be at least 300, at least 400, at least 500, at least 1000, at least 1500 or at least 2000 nucleotides in length. The insulator may function either as an enhancer-blocker or a barrier, or both.

The DNA cassette may be an expression cassette. The DNA cassette may be a eukaryotic expression cassette e.g. a mammalian expression cassette or a plant expression cassette. The DNA cassette may be a prokaryotic expression cassette e.g. a bacterial expression cassette.

The DNA cassette may further comprise a reporter gene. The reporter gene may be an eGFP reporter gene or a luciferase reporter gene.

The DNA cassette may additionally comprise a sequence aiding protein expression, e.g. a cap-independent translation element.

The DNA cassette may comprise a gene of interest (GOI). The DNA cassette may comprise the sequence for at least two, three, four, or five genes of interest.

The DNA cassette may comprise (or encode) a repair template (or editing template). The repair template (or editing template) may be for use in CRISPR-Cas mediated homology directed repair (HDR). The DNA cassette may encode a CRISPR guide RNA.

The DNA cassette may further comprise a LoxP sequence, preferably two LoxP sequences. The two LoxP sequences may be oriented in the same direction. In this case, the DNA sequence between the two LoxP sequences may be excised as a circular loop of DNA. The two LoxP sequences may be oriented in the opposite directions. In this case, the DNA sequence between the two LoxP sequences may be inverted. Preferably, the two LoxP sequences are in the same orientation (i.e. the same direction) in the cassette. One LoxP sequence may be at a first end of the cassette and the other LoxP sequence may be at a second end of the cassette. One LoxP sequence may be upstream and the other LoxP sequence may be downstream of the other sequence components of the cassette. The DNA cassette may be a functional cassette i.e. it may have all sequence elements required for expression of a coding sequence.

Any of the sequence components described above in relation to the DNA cassette may be comprised in a single cassette sequence or split across two or more cassette sequences. For example, a coding sequence may be comprised in a single cassette sequence or split across two or more cassette sequences. A promoter may be comprised in a single cassette sequence or split across two or more cassette sequences.

The first cassette sequence may comprise a promoter and the second cassette sequence may comprise a coding sequence.

The single-stranded DNA (ssDNA) cassette may comprise a nuclease-resistant nucleotide at the 5' end of the ssDNA cassette or 5' of the ssDNA cassette, and a nuclease-resistant nucleotides at the 3'-end of the ssDNA cassette or 3' of the ssDNA cassette, wherein the cassette comprises at least 100 nucleotides.

The single-stranded DNA (ssDNA) cassette may comprise at least 3 nuclease-resistant nucleotides at the 5' end of the ssDNA cassette or 5' of the ssDNA cassette, and at least 3 nuclease-resistant nucleotides at the 3'-end of the ssDNA cassette or 3' of the ssDNA cassette, optionally wherein the ssDNA cassette comprises at least 100 nucleotides.

The single-stranded DNA (ssDNA) cassette may comprise at least 5 nuclease-resistant nucleotides at the 5' end of the ssDNA cassette or 5' of the ssDNA cassette, and at least 5 nuclease-resistant nucleotides at the 3'-end of the ssDNA cassette or 3' of the ssDNA cassette, optionally wherein the ssDNA cassette comprises at least 100 nucleotides.

The single-stranded DNA (ssDNA) cassette may comprise x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1, optionally wherein the ssDNA cassette comprises at least 100 nucleotides.

x and y may be independently selected from at least 1, at least 2, at least 3, at least 4 and at least 5. x and y may be different or the same. Preferably x and y are both at least 3. x and y may both be at least 5. x may be 3 and y be 1 and vice versa. x may be 3 and y be 5 and vice versa.

The linear DNA product may be resistant to 3'-end exonuclease digestion (e.g. by exonuclease III) and/or 5'-end exonuclease digestion (e.g. by exonuclease VIII). The protected DNA may be resistant to 3'-end exonuclease digestion (e.g. by exonuclease III, exonuclease T and/or exonuclease VII) and/or 5'-end exonuclease digestion (e.g. by exonuclease VIII, exonuclease VII, RecJf, RecJ, T7 exonuclease, Lambda exonuclease and/or T5 exonuclease).

The step of incubating the single contiguous aqueous volume to generate the linear DNA product (e.g. a closed linear DNA product, or a partially closed linear DNA product) may comprise generating the linear portion of the double-stranded DNA molecule by digesting the double-stranded DNA molecule with the endonuclease.

The step of incubating the single contiguous aqueous volume may be performed under conditions that promote appending (or linking) of the third and fourth adaptor molecules to the linear double-stranded region to produce the linear DNA product. The appending may be performed by creating a covalent link between the third and/or fourth adaptor molecule and the end(s) of the linear double-stranded region (or linear portion of the double-stranded DNA molecule).

The step of incubating the single contiguous aqueous volume may be performed under conditions that promote digestion of the double-stranded DNA molecule to produce the linear portion of the double-stranded DNA molecule. The digestion of the double-stranded DNA molecule to produce the linear portion of the double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion. The digestion may be performed for at least 10 min, at least 30 min, or at least 1 hour.

The step of incubating the single contiguous aqueous volume may be performed at about 37°C for at least 10 hours, preferably at least 20 hours.

The step of incubating the single contiguous aqueous volume may be performed under conditions that promote ligation of the linear double-stranded region to the third and fourth adaptor molecules. The ligation may be at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% efficient. For example, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% of the linear double-stranded regions (or the portions of the double-stranded DNA molecules) may be incorporated into closed linear DNA products. Preferably, the ligation is at least 15% or at least 25% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining unprotected DNA molecules and adaptor molecules excess.

For example, the amplified DNA products generated by rolling circle amplification is first quantified so that the amount of the amplified DNA products used as the starting material during the digestion/ligation reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the linear double-stranded region to the third and fourth adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C. Preferably, the second temperature is 14°C-18°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is about 37°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous volume does not deviate by more than 5°C, preferably by not more than 3°C, even more preferably not more than 1°C. Preferably, the constant temperature is a temperature in a range of 33°C-40°C.

The method of producing a linear DNA product may comprise the steps of:
(a) amplifying a first linear dsDNA template molecule to generate a first amplified DNA product, wherein the first DNA template molecule comprises at least one endonuclease target sequence and wherein the first amplified DNA product is a first concatemer, and amplifying a second linear dsDNA template molecule to generate a second amplified DNA product, wherein the second DNA template molecule comprises at least one endonuclease target sequence and wherein the second amplified DNA product is a second concatemer;
(b) contacting the first amplified DNA product, the second amplified DNA product, an endonuclease, a ligase, a first hairpin adaptor, and a second hairpin adaptor to form a single contiguous aqueous volume;
(c) incubating the single contiguous aqueous volume to generate a closed linear DNA template molecule, wherein the closed linear DNA template molecule comprises a linear double-stranded region, wherein the linear double-stranded region comprises a linear portion of the first amplified DNA product and a linear portion of the second amplified DNA product, and wherein the closed linear DNA template molecule comprises at least one endonuclease target sequence;
(d) amplifying the closed linear DNA template molecule to generate a double-stranded DNA molecule, wherein the closed linear DNA template molecule is amplified by rolling circle amplification;
(e) contacting the double-stranded DNA molecule with an endonuclease, a ligase and third and fourth adaptor molecules to form a single contiguous aqueous volume; and
(f) incubating the single contiguous aqueous volume to generate the linear DNA product of improved resistance to nuclease digestion.

The invention also provides a method for producing a linear deoxyribonucleic acid (DNA) product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification in the presence of at least one type of a nuclease-resistant nucleotide; and
(d) contacting the double-stranded DNA molecule with at least one endonuclease to generate the linear double-stranded DNA product, wherein the linear double-stranded DNA product has increased resistance to nucleases, such as exonucleases.

The linear double-stranded DNA product produced by the methods of the invention may comprise a sense strand and an antisense strand. The linear double-stranded DNA product may comprise one or more protected nucleotides (e.g. phosphorothioated nucleotides) at internal positions in each strand.

As used herein the term "protected nucleotide" or "nuclease-resistant nucleotide" is intended to encompass any type of molecule that provides or enhances resistance to nuclease digestion (especially exonuclease digestion). Although the linear double-stranded DNA product is mostly described herein as comprising phosphorothioated nucleotides, the skilled person would appreciate that the linear double-stranded DNA product may instead comprise any molecules that provide resistance to nuclease digestion (e.g. exonuclease III digestion). For example, the linear double-stranded DNA product may comprise nuclease resistant nucleotides i.e. modified nucleotides that provide or increase resistance to nucleases (e.g. exonucleases). The linear double-stranded DNA product may comprise a peptide, polypeptide or protein that provides or increases resistance to nucleases (e.g. exonucleases) digestion. The linear double-stranded DNA product may comprise 2'-O-methyl nucleotides or 2'-O-methoxyethyl (MOE) nucleotides.

The internal positions may be any positions in the linear double-stranded DNA product other than the last nucleotide on the 3'-end and the 5'-end of the sense and antisense strands. The internal positions may be located at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA product.

The linear double-stranded DNA product may comprise a cassette. The linear double-stranded DNA product may comprise a single cassette. The term "single cassette" as used herein is intended to encompass a linear double-stranded DNA product that does not comprise or consist of a plurality of cassettes. That is to say that the linear double-stranded DNA product comprises only a single cassette, which may comprise a single coding sequence of a gene of interest. For example, the single cassette may not comprise or consist of a plurality of tandem repeat sequences, and/or concatemeric DNA.

The cassette may comprise a coding sequence. The coding sequence may encode a gene of interest, for example a gene encoding a protein. For protein expression, the cassette comprising a coding sequence may be ligated into a vector or a plasmid comprising at least a portion of a promoter so that the portion of the promoter is operably linked to the coding sequence (e.g. the promoter may be upstream of the coding sequence). The vector or plasmid may further comprise a guide RNA (gRNA) of the Clustered Regularly Interspaced Short Palindromic Repeats (CRISPR) system. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The location of the protected (e.g. phosphorothioated) nucleotides in the linear double-stranded DNA product may be such that the cassette is protected from nuclease (e.g. exonuclease III) digestion. Thus, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The term "*5'-end nucleotide of the cassette"* as used herein is intended to encompass the 5'-end nucleotide of each strand of the cassette. Thus, in the linear double-stranded DNA product, the cassette typically comprises a 5'-end nucleotide of the sense strand and a 5'-end nucleotide of the antisense strand.

The term *"3'-end nucleotide of the cassette"* as used herein is intended to encompass the 3'-end nucleotide of each strand of the cassette. Thus, in the linear double-stranded DNA product, the cassette typically comprises a 3'-end nucleotide of the sense strand and a 3'-end nucleotide of the antisense strand.

The term *"outside of the cassette"* as used herein is intended to encompass any nucleotides which are not part of the cassette. This includes any nucleotides that are comprised in the linear double-stranded DNA product and which also do not form part of the cassette. The term *"N nucleotides outside of the cassette"* or *"N nucleotides away from the cassette"* is intended to describe nucleotides that are located N nucleotides from the end of the cassette towards the end of the linear double-stranded DNA product. For example, the term "2 *nucleotides outside of the cassette"* in the context of internal positions of nucleotides is meant to describe a nucleotide that is outside of the cassette and that is two nucleotides away from the last nucleotide of the cassette.

The internal positions may not be located between the second and penultimate nucleotide of the cassette. The internal positions may be any position in the linear double-stranded DNA product other than the last nucleotide on the 3'-end and the 5'-end of the sense and antisense strands and other than nucleotides located between the second and penultimate nucleotide of the cassette.

The linear double-stranded DNA product may comprise at least 2, 3, 4, 5, 6, 7, 8, 9 or 10 protected (e.g. phosphorothioated) nucleotides at internal positions in each strand. Preferably, the linear double-stranded DNA product comprises at least 2 protected (e.g. phosphorothioated) nucleotides at internal positions in each strand.

The linear double-stranded DNA product may comprise a sense strand and an antisense strand, wherein the linear double-stranded DNA product comprises a single cassette and at least two nuclease-resistant nucleotides (i.e. protected nucleotides) at internal positions in each strand, wherein the at least two nuclease-resistant nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The linear double-stranded DNA product may comprise a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The location of phosphorothioated nucleotides in the sense strand of the linear double-stranded DNA product may be such that the one or more phosphorothioated nucleotides are located upstream of the cassette (i.e. towards the 5'-end of the sense strand of the DNA product).

In the sense strand of the linear double-stranded DNA product:
(a) one of the at least two phosphorothioated nucleotides may be the 5'-end nucleotide of the cassette; and/or
(b) one of the at least two phosphorothioated nucleotides may be in a first region of the sense strand, wherein the first region of the sense strand is 5' of the cassette.

The location of phosphorothioated nucleotides in the sense strand of the linear double-stranded DNA product may be such that the one or more phosphorothioated nucleotides are located downstream of the cassette (i.e. towards the 3'-end of the sense strand of the DNA product).

In the sense strand of the linear double-stranded DNA product:
(a) one of the at least two phosphorothioated nucleotides may be the 3'-end nucleotide of the cassette; and/or
(b) one of the at least two phosphorothioated nucleotides may be in a second region of the sense strand, wherein the second region of the sense strand is 3' of the cassette.

The location of phosphorothioated nucleotides in the antisense strand of the linear double-stranded DNA product may be such that the one or more phosphorothioated nucleotides are located upstream of the cassette (i.e. towards the 5'-end of the antisense strand of the DNA product).

In the antisense strand of the linear double-stranded DNA product:
(a) one of the at least two phosphorothioated nucleotides may be the 5'-end nucleotide of the cassette; and/or
(b) one of the at least two phosphorothioated nucleotides may be in a first region of the antisense strand, wherein the first region of the antisense strand is 5' of the cassette.

The location of phosphorothioated nucleotides in the antisense strand of the linear double-stranded DNA product may be such that the one or more phosphorothioated nucleotides are located downstream of the cassette (i.e. towards the 3'-end of the antisense strand of the DNA product).

In the antisense strand of the linear double-stranded DNA product:
(a) one of the at least two phosphorothioated nucleotides may be the 3'-end nucleotide of the cassette; and/or
(b) one of the at least two phosphorothioated nucleotides may be in a second region of the antisense strand, wherein the second region of the antisense strand is 3' of the cassette.

The location of phosphorothioated nucleotides in both the sense and the antisense strand of the linear double-stranded DNA product may be such that at least one phosphorothioated nucleotide is located downstream of the cassette and at least one phosphorothioated nucleotide is located upstream of the cassette in each strand.

In the linear double-stranded DNA product:
(a) in the sense strand one of the at least two phosphorothioated nucleotides may be the 5'-end nucleotide of the cassette, and/or one of the at least two phosphorothioated nucleotides may be in a first region of the sense strand, wherein the first region of the sense strand is 5' of the cassette;
(b) in the sense strand one of the at least two phosphorothioated nucleotides may be the 3'-end nucleotide of the cassette, and/or one of the at least two phosphorothioated nucleotides may be in a second region of the sense strand, wherein the second region of the sense strand is 3' of the cassette;
(c) in the antisense strand one of the at least two phosphorothioated nucleotides may be the 5'-end nucleotide of the cassette, and/or one of the at least two phosphorothioated nucleotides may be in a first region of the antisense strand, wherein the first region of the antisense strand is 5' of the cassette; and
(d) in the antisense strand one of the at least two phosphorothioated nucleotides may be the 3'-end nucleotide of the cassette, and/or one of the at least two phosphorothioated nucleotides may be in a second region of the antisense strand, wherein the second region of the antisense strand is 3' of the cassette.

The term *"first region of the sense strand"* as used herein is intended to encompass the part of the sense strand of the linear double-stranded DNA product that is between the 5'-end of the linear double-stranded DNA product and the first 5' nucleotide of the cassette in the sense strand. The term *"first region of the antisense strand"* is intended to encompass the part of the antisense strand of the linear double-stranded DNA product that is between the 5'-end of the linear double-stranded DNA product and the first 5' nucleotide of the cassette in the antisense strand. The term *"second region of the sense strand"* is intended to encompass the part of the sense strand of the linear double-stranded DNA product that is between the 3'-end of the linear double-stranded DNA product and the first 3' nucleotide of the cassette in the sense strand. The term *"second region of the antisense strand"* is intended to encompass the part of the antisense strand of the linear double-stranded DNA product that is between the 3'-end of the linear double-stranded DNA product and the first 3' nucleotide of the cassette in the antisense strand.

The linear double-stranded DNA product may comprise a plurality of the phosphorothioated nucleotides upstream (i.e. towards the 5'-end of the sense strand of the DNA product) of the cassette. For example, the linear double-stranded DNA product may comprise at least 2, 3, 4, 5, 6, 7, 8, 9 or 10 phosphorothioated nucleotides upstream of the cassette. Preferably, at least 2 phosphorothioated nucleotides upstream of the cassette. Thus, the location of phosphorothioated nucleotides in the sense strand of the linear double-stranded DNA product may be such that at least two phosphorothioated nucleotides are located upstream of the cassette.

In the sense strand of the linear double-stranded DNA product:
(a) one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
(b) the at least two phosphorothioated nucleotides may be in a first region of the sense strand, wherein the first region of the sense strand is 5' of the cassette.

The linear double-stranded DNA product may comprise a plurality of the phosphorothioated nucleotides downstream (i.e. towards the 3-end of the DNA product) of the cassette. For example, the linear double-stranded DNA product may comprise at least 2, 3, 4, 5, 6, 7, 8, 9 or 10 phosphorothioated nucleotides downstream of the cassette, preferably at least 2 phosphorothioated nucleotides downstream of the cassette. Thus, the location of phosphorothioated nucleotides in the sense strand of the linear double-stranded DNA product may be such that at least two phosphorothioated nucleotides are located downstream of the cassette.

In the sense strand of the linear double-stranded DNA product:
(a) one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
(b) the at least two phosphorothioated nucleotides may be in a first region of the sense strand, wherein the first region of the sense strand is 3' of the cassette.

The location of phosphorothioated nucleotides in the antisense strand of the linear double-stranded DNA product may be such that at least two phosphorothioated nucleotides are located upstream of the cassette (i.e. towards the 5'-end of the antisense strand of the DNA product).

In the antisense strand of the linear double-stranded DNA product:
(a) one of the at least two phosphorothioated nucleotides is the 5'-end nucleotide of the cassette and one of the at least two phosphorothioated nucleotides is in a first region of the antisense strand, wherein the first region of the antisense strand is 5' of the cassette; or
(b) the at least two phosphorothioated nucleotides are in a first region of the antisense strand, wherein the first region of the antisense strand is 5' of the cassette.

The location of phosphorothioated nucleotides in the antisense strand of the linear double-stranded DNA product may be such that at least two phosphorothioated nucleotides are located downstream of the cassette (i.e. towards the 3'-end of the antisense strand of the DNA product).

In the antisense strand of the linear double-stranded DNA product:
(a) one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
(b) the least two phosphorothioated nucleotides may be in a second region of the antisense strand, wherein the second region of the antisense strand is 3' of the cassette.

Each strand of the linear double-stranded DNA product may comprise a plurality of phosphorothioated nucleotides. For example, the linear double-stranded DNA product may comprise at least 2, 3, 4, 5, 6, 7, 8, 9 or 10 phosphorothioated nucleotides upstream and downstream of the cassette. The location of phosphorothioated nucleotides in both the sense and the antisense strand of the linear double-stranded DNA product may be such that the at least two phosphorothioated nucleotides are located downstream of the cassette and at least two phosphorothioated nucleotides are located upstream of the cassette in each strand.

In the linear double-stranded DNA product:
(a) in the sense strand:
   i. one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
   ii. the at least two phosphorothioated nucleotides may be in a first region of the sense strand, wherein the first region of the sense strand is 5' of the cassette;
(b) in the sense strand:
   i. one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
   ii. the at least two phosphorothioated nucleotides may be in a second region of the sense strand, wherein the second region of the sense strand is 3' of the cassette;
(c) in the antisense strand:
   i. one of the at least two phosphorothioated nucleotides may be the 5'-end nucleotide of the cassette and one of the at least two phosphorothioated nucleotides may be in a first region of the antisense strand, wherein the first region of the antisense strand is 5' of the cassette; or
   ii. the at least two phosphorothioated nucleotides may be in a first region of the antisense strand, wherein the first region of the antisense strand is 5' of the cassette; and
(d) in the antisense strand:
   i. one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
   ii. the at least two phosphorothioated nucleotides may be in a second region of the antisense strand, wherein the second region of the antisense strand is 3' of the cassette.

The amount of phosphorothioated nucleotides at internal positions in each strand may depend on the length of the linear double-stranded DNA product. Thus, the linear double-stranded DNA product may comprise phosphorothioated nucleotides at a ratio of the phosphorothioated nucleotides to total nucleotides that is at least 0.0001, at least 0.0025, at least 0.01 at least 0.025, at least 0.05, at least 0.075, at least 0.10, at least 0.12 at least 0.15, at least 0.25, at least 0.35, at least 0.5, or at least 0.75. The linear double-stranded DNA product may comprise phosphorothioated nucleotides at a ratio of the phosphorothioated nucleotides to total nucleotides that is less than 1, less than 0.9, less than, 0.8, less than 0.65, less than 0.5, less than 0.4, less than 0.3, less than 0.2, less than 0.1, less than 0.075, or less than 0.05. The linear double-stranded DNA product may comprise phosphorothioated nucleotides at a ratio of the phosphorothioated nucleotides to total nucleotides that is between 0.0001-1, between 0.0025-0.75, between 0.025-0.65, between 0.025-0.15, or between 0.25-0.50. Preferably, a ratio of the phosphorothioated nucleotides to total nucleotides is between 0.025-0.15. The linear double-stranded DNA product may comprise phosphorothioated nucleotides at a ratio of the phosphorothioated nucleotides to total nucleotides that is between 0.005-0.3, between 0.0075-0.2, between 0.01-0.15, between 0.01-0.10, between 0.02-0.08, between 0.03-0.07, between 0.04-0.06 or between 0.05-0.075. Preferably, a ratio of the phosphorothioated nucleotides to total nucleotides is between 0.01-0.10. Preferably, a ratio of the phosphorothioated nucleotides to total nucleotides is about 0.02, about 0.025, about 0.05, about 0.075, about 0.08, about 0.10, about 0.12, about 0.15, or about 0.25. More preferably still, a ratio of the phosphorothioated nucleotides to total nucleotides is about 0.025 or about. 0.05.

### 4. Further optional method steps

The invention provides a method for producing a linear DNA product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product of improved resistance to nuclease digestion.

The linear DNA product may be a closed linear DNA product. Thus, the method for producing a closed DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the third adaptor molecule and closed at a second end by the fourth adaptor molecule.

The linear DNA product may be a linear DNA product comprising nuclease-resistant nucleotides. Thus, the method for producing a linear DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product, wherein the linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third and fourth adaptor molecules are nucleic acid molecules that comprise one or more nuclease-resistant nucleotides.

The linear DNA product may be a partially closed DNA product. The partially closed DNA product may be a partially closed linear DNA product. Thus, the method for producing a partially closed DNA product may comprise:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third adaptor molecule is a nucleic acid molecule that comprise one or more nuclease-resistant nucleotides, and wherein the linear double-stranded region is closed at the second end by the fourth adaptor molecule.

The invention provides a method for producing a linear DNA product of improved resistance to nuclease digestion, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification in the presence of at least one type of a nuclease-resistant nucleotide; and
(d) contacting the double-stranded DNA molecule with at least one endonuclease to generate the linear double-stranded DNA product, wherein the linear double-stranded DNA product has increased resistance to nucleases, such as exonucleases.

The methods may further comprise, after the step of incubating the single contiguous aqueous volume, or after a step of contacting the double-stranded DNA molecule with at least one endonuclease to generate the linear double-stranded DNA product, a step of purification of the linear DNA product.

The methods may further comprise, after the step of incubating the single contiguous aqueous volume, or after a step of contacting the double-stranded DNA molecule with at least one endonuclease to generate the linear double-stranded DNA product, a step of nuclease digestion. The nuclease digestion may be exonuclease digestion, such as exonuclease I and/or exonuclease III digestion. The step of nuclease digestion may take place before or after the step of purification. This step allows for removal of any double-stranded DNA molecules and/or adaptor molecules which have not been used in the course of performing the methods. The nuclease digestion may also or alternatively be performed before the step of amplification.

The step of nuclease digestion may be performed at a temperature of 5-90°C, 10-80°C, 15-70°C, 20-60°C, 25-50°C, 30-45°C or 35-40°C. The step of nuclease digestion may be performed for at least 10, at least 20, at least 30, at least 40, at least 50, or at least 60 min. The step of nuclease digestion may be performed at two different temperatures. For example, the step of nuclease digestion may be performed at 15-40°C for 10-60 minutes followed by a temperature of 60-90°C for 10-30 min. The higher temperature typically inactivates the nuclease (e.g. exonuclease). Thus, the method may further comprise a step of inactivating the nuclease (e.g. exonuclease). The step of inactivating the nuclease may be performed at 37°C for at least 30 min, for example 2 hours. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The methods may further comprise (after the steps of amplification and before the step of contacting the double-stranded DNA molecule with an endonuclease) a step of heat-deactivation. The step of heat-deactivation may be performed under conditions sufficient to inactivate the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The methods may comprise amplifying linear dsDNA template molecules (e.g. first and second linear dsDNA template molecules) to generate amplified DNA products (e.g. first and second amplified DNA products), wherein the linear dsDNA template molecules comprise at least one cleavable target sequence (e.g. endonuclease target sequence).

The method may further comprise a step of re-amplification of the linear DNA product. The step of re-amplification may be performed after the linear DNA product has been generated. If the step of re-amplification is performed, the third and fourth adaptor molecules may be hairpin adaptor molecules. If the step of re-amplification is performed, the endonuclease target sequences of the third and fourth adaptor molecules may be different to the endonuclease target sequences of the first and second hairpin adaptors. If the step of re-amplification is performed, a step of purification may be performed after the linear DNA product is produced. The step of re-amplification is particularly useful to generate large amounts of dsDNA template molecules and/or to generate long-term storage stocks.

### 5. Uses and applications

The linear DNA products produced by the methods of the invention find application in different fields of biotechnology and pharmacology.

The linear DNA products produced by the methods of the invention are particularly suitable for use in therapy. The invention provides the use of the linear DNA product produced by the methods described herein in therapy. The linear DNA product may encode a sequence of a therapeutic protein, a part of a vaccine, or an element of a genetic engineering mechanism, which is used to treat a disease or infection in a subject. The invention further provides the linear DNA product produced by the methods described herein for use as a medicament. The invention also provides the use of a linear DNA product produced by the methods described herein for the manufacture of a medicament for treating a disease. The invention further provides the linear DNA product produced by the methods described herein for use in treating a disease.

The linear DNA product produced by the methods described herein may be used to treat any disease or disorder. For example, the linear DNA product may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product is used to treat a monogenic disorder. For example, the linear DNA product, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The invention provides the use of the linear DNA product produced by the methods described herein for nuclear expression of a gene of interest.

The invention provides the use of the linear DNA product produced by the methods described herein for expression of RNA in a cell. The RNA may be miRNA or IncRNA.

The linear DNA product produced by the methods described herein is very simple in nature (e.g. no bacterial backbone), which means that it is typically easy to work with. The pure and simple nature of the product described herein makes it particularly suitable for use in cell therapy. For example, a cell comprising the linear DNA product may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the linear DNA product produced by the methods described herein for use in cell therapy.

Preferably, cell therapy is ex-vivo cell therapy. The cell may be an animal cell, preferably mammal cell, such as human cell.

The invention provides the use of a linear DNA product produced by the methods described herein in the production of a CAR-T cell.

The invention provides a method for producing a genetically engineered CAR-T cell comprising: (a) introducing the linear DNA product produced by the methods described herein into a T cell; and (b) expressing a gene of interest encoded by the linear DNA product. Preferably, the gene of interest is a tumour-specific CAR.

The method may further comprise, before step (a) (i.e. introducing the linear DNA product into a T cell), a step of removing mononuclear cells from a patient. Preferably, the step of removing is performed using leukapheresis. Preferably the mononuclear cells are T cells. The method may further comprise, after step (b) (i.e. expressing a gene of interest), a step of returning the genetically engineered cells to the patient.

The invention also provides an engineered T cell obtainable by any methods described herein. The engineered T cell may be suitable for use in CAR T-cell therapy.

The invention also provides the use of the linear DNA product produced by the methods described herein in gene editing.

The invention also provides the use of the linear DNA product produced by the methods described herein in gene therapy.

The linear DNA product produced by the methods described herein is particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The linear DNA product may comprise a gene sequence encoding any component of the CRISPR machinery. The linear DNA product may encode all components of the CRISPR machinery.

The linear DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1 or Mad7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000. 15000, or 20000 base pairs long. The linear DNA product encoding the repair template may be delivered to a cell by a nanoparticle, a non-viral vector or a viral vector, or without the aid of any carrier. The linear DNA product encoding the repair template may be delivered to a cell by electroporation. The linear DNA product encoding the repair template may be delivered to a cell by hydrodynamic needle.

The linear DNA product may comprise (or further comprise) a gene sequence encoding a nuclease protein of a CRISPR system (e.g. Cas9, Cpf1 or Mad7), and/or a guide RNA. The linear DNA product may comprise (or further comprise) a gene sequence encoding a nuclease protein of a CRISPR system (e.g. Cas9, Cpf1, or Mad7). The linear DNA product may comprise (or further comprise) a gene sequence encoding a guide RNA. The linear DNA product may comprise (or further comprise) a gene sequence encoding a nuclease protein of a CRISPR system (e.g. Cas9, Cpf1, or Mad7) and a guide RNA. The linear DNA product may comprise (or further comprise) a gene sequence encoding a genomic target to be modified (e.g. a spacer). The linear DNA product may be ligated to a vector. The vector may comprise a sequence of some of the components of the CRISPR system. The vector may comprise a sequence of guide RNA or a sequence of a part of the guide RNA. If the vector comprises a sequence of a part of the guide RNA, the linear DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The linear DNA product may encode the nuclease of the CRISPR system and guide RNA. One linear DNA product may encode the nuclease of the CRISPR system, and the other linear DNA product may encode guide RNA.

The linear DNA product may comprise (or further comprise) a gene sequence encoding a nuclease protein of a CRISPR system (e.g. Cas9, Cpf1 or Mad7), and/or a gene sequence encoding a DNA polymerase, a reverse transcriptase, or an exonuclease. The linear DNA product may comprise (or further comprise) a gene sequence encoding a DNA polymerase, a reverse transcriptase or an exonuclease. The linear DNA product may comprise (or further comprise) a gene sequence encoding a nuclease protein of a CRISPR system (e.g. Cas9, Cpf1, or Mad7) and a gene sequence encoding a DNA polymerase, a reverse transcriptase or an exonuclease.

The linear DNA product may be for use in CRISPR-Cas mediated repair by recombination, homology-directed repair, or non-homologous end joining.

If the nuclease of the CRISPR system and the guide RNA are encoded by a different linear DNA product, they may be part of a different or the same delivery mechanism. For example, the linear DNA product encoding the nuclease of the CRISPR system may be delivered to a cell by a first nanoparticle, non-viral vector or viral vector, whereas the linear DNA product encoding the guide RNA may be delivered to a cell by a second nanoparticle, a non-viral vector, or viral vector. For example, the linear DNA product encoding the nuclease of the CRISPR system and the linear DNA product encoding the guide RNA may be delivered to a cell by the same nanoparticle, non-viral vector or viral vector.

If the nuclease of the CRISPR system and the guide RNA are encoded by the same linear DNA product (or by a vector that comprises the linear DNA product) they may be part of the same delivery mechanism. For example, the linear DNA product, or the vector, encoding the nuclease of the CRISPR system and the guide RNA may be delivered to a cell by a nanoparticle, a non-viral vector or a viral vector.

The linear DNA product encoding the nuclease of the CRISPR system and the linear DNA product encoding the guide RNA may be delivered to a cell by electroporation. The linear DNA product encoding the nuclease of the CRISPR system and the linear DNA product encoding the guide RNA may be delivered to a cell by hydrodynamic needle.

Thus, the invention provides the linear DNA product produced by the methods described herein for use in CRISPR system delivery to a cell. The invention provides a method of delivering the CRISPR system to a cell, comprising contacting the linear DNA product produced by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

The linear DNA product produced by the methods described herein may be used in transcription, to generate RNA, preferably mRNA, in vitro or in vivo.

The linear DNA product may comprise (or further comprise) a sequence encoding for self-amplifying mRNA (SAM). The linear DNA product may comprise (or further comprise) a sequence encoding for tRNA. The linear DNA product may comprise (or further comprise) a sequence encoding for circular mRNA (or circRNA).

The invention provides a use of a linear DNA product produced by the methods described herein in the production of a viral or non-viral delivery system.

The invention provides a use of a linear DNA product in the production of a viral or non-viral delivery system.

The invention provides a viral or non-viral delivery system comprising a linear DNA product produced by the methods described herein.

Methods of producing viral vectors, such as AAV vectors, are known in the art. The most widely used method involves the co-transfection of HEK293 by three bacterial plasmids. The first plasmid encodes the *Rep* and *Cap* element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli;* 3) the incorporation of the plasmid backbone into the viral capsid (e.g. AAV capsid) might be challenging (e.g. issues with antibiotic resistance markers). Methods of producing viral vectors may be performed using other cell lines. For example, a cell line suitable for production of viral vectors may be a Vero cell, or any other stable cell line. Together these represent the main bottleneck in viral vector production (e.g. AAV production). Thus, the linear DNA product produced by the methods described herein is suitable for use in production of viral vectors. The linear DNA product overcomes the above issues with plasmid vectors.

The invention provides a method of producing a viral vector, the method comprising introducing the linear DNA product produced by the methods described herein into a cell under conditions such that the viral vector is produced. The linear DNA product may encode at least one element required for the production of the viral vector. For example, the linear DNA product may encode Rep and/or Cap elements. The linear DNA product may encode the helper plasmid elements. The linear DNA product may encode Rep, Cap and helper plasmid elements. The linear DNA product may encode a transgene. The method may be an in vivo or in vitro method. The cell may be an animal cell, preferably mammalian cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the linear DNA product) to a cell, comprising contacting the viral vector produced by the methods described herein with the cell. The cell may be an animal cell, preferably mammalian cell, such as human cell.

The invention also provides a cell obtainable by the methods described herein.

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the linear DNA product produced by the methods described herein is suitable for use in production of non-viral vectors. The linear DNA product produced or obtainable by the methods of the invention overcomes the issues of using plasmid vectors in non-viral vector preparation. For example, the linear DNA product, unlike plasmid DNA, does not comprise a bacterial backbone, allowing more transgene copies per mg of DNA. In addition, the linear DNA product does not comprise antibiotic resistant genes and bacterial contaminants. Moreover, the linear DNA product provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the linear DNA product with the cell. The cell may be an animal cell, preferably mammalian cell, such as human cell.

The invention also provides a cell obtainable by the methods described herein.

The linear DNA product produced by the methods described herein is particularly suitable for use in vaccine production. A vaccine may comprise a linear DNA product produced by the methods described herein. Alternatively, the linear DNA product may be used to produce a vaccine, preferably an mRNA-based vaccine. For example, vaccines such as the BioNTech and Moderna mRNA vaccines against severe acute respiratory syndrome coronavirus 2 (SARS-CoV-2). The linear DNA product may be used to produce a seasonal, pandemic, prophylactic or therapeutic vaccine. The linear DNA product produced by the methods described herein is particularly suitable for use in personalised vaccine production.

Thus, the invention provides the use of the linear DNA product produced by the methods described herein in the production of a vaccine.

The linear DNA product may encode an antigen, which may cause an immune response in a subject. The subject may be human. Preferably, the antigen is encoded on the DNA cassette. The linear DNA product may encode a neoantigen, which may cause an immune response in a subject. Preferably, the neoantigen is encoded on the DNA cassette.

### 6. Kits

The invention provides a kit comprising components required to carry out the methods described herein.

The kit may comprise:
(a) a strand displacing polymerase;
(b) an endonuclease;
(c) a ligase; and
(d) optionally first and second hairpin adaptors.

The kit may comprise:
(a) a strand displacing polymerase;
(b) an endonuclease;
(c) a ligase;
(d) optionally first and second hairpin adaptors; and
(e) optionally third and fourth adaptor molecules.

Preferably, the first and second hairpin adaptors are different to the third and fourth adaptor molecules. The adaptors are described in other sections of the present disclosure.

The strand-displacing polymerase may be Phi29 DNA polymerase or a mutant thereof which retains functionality (e.g. QualiPhi^{®}, a chimeric form of Phi29 DNA polymerase from 4basebio).

The kit may further comprise an exonuclease e.g. exonuclease I, exonuclease III and/or exonuclease VIII.

The endonuclease may be any endonuclease described herein. Preferably, the endonuclease is a Type IIS restriction enzyme.

The ligase may a DNA ligase, such as a T4 DNA ligase, T7 DNA ligase, mammalian DNA ligase I, III and IV; Taq DNA ligase, Tth DNA ligase, or E. coli DNA ligase.

The foregoing description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

The present invention is also defined in the following clauses.
1. A method for producing a closed linear deoxyribonucleic acid (DNA) product, wherein the method comprises:
   (a) providing a linear double stranded (ds)DNA template molecule;
   (b) contacting the linear dsDNA template molecule with a first hairpin adaptor and a second hairpin adaptor to generate a closed linear DNA template molecule, wherein the closed linear DNA template molecule comprises at least one endonuclease target sequence;
   (c) amplifying the closed linear DNA template molecule to generate a double-stranded DNA molecule, wherein the closed linear DNA template molecule is amplified by rolling circle amplification;
   (d) contacting the double-stranded DNA molecule with an endonuclease, a ligase and third and fourth adaptor molecules to form a single contiguous aqueous volume; and
   (e) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the third adaptor molecule and closed at a second end by the fourth adaptor molecule.
2. A method for producing a linear deoxyribonucleic acid (DNA) product, wherein the method comprises:
   (a) providing a linear double stranded (ds)DNA template molecule;
   (b) contacting the linear dsDNA template molecule with a first hairpin adaptor and a second hairpin adaptor to generate a closed linear DNA template molecule, wherein the closed linear DNA template molecule comprises at least one endonuclease target sequence;
   (c) amplifying the closed linear DNA template molecule to generate a double-stranded DNA molecule, wherein the closed linear DNA template molecule is amplified by rolling circle amplification;
   (d) contacting the double-stranded DNA molecule with an endonuclease, a ligase and third and fourth adaptor molecules to form a single contiguous aqueous volume; and
   (e) incubating the single contiguous aqueous volume to generate the linear DNA product, wherein the linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third and fourth adaptor molecules are nucleic acid molecules that comprise one or more nuclease-resistant nucleotides.
3. A method for producing a partially closed deoxyribonucleic acid (DNA) product, wherein the method comprises:
   (a) providing a linear double stranded (ds)DNA template molecule;
   (b) contacting the linear dsDNA template molecule with a first hairpin adaptor and a second hairpin adaptor to generate a closed linear DNA template molecule, wherein the closed linear DNA template molecule comprises at least one endonuclease target sequence;
   (c) amplifying the closed linear DNA template molecule to generate a double-stranded DNA molecule, wherein the closed linear DNA template molecule is amplified by rolling circle amplification;
   (d) contacting the double-stranded DNA molecule with an endonuclease, a ligase and third and fourth adaptor molecules to form a single contiguous aqueous volume; and
   (e) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third adaptor molecule is a nucleic acid molecule that comprise one or more nuclease-resistant nucleotides, and wherein the linear double-stranded region is closed at the second end by the fourth adaptor molecule.
4. A method for producing a linear double-stranded DNA product, wherein the method comprises:
   (a) providing a linear double stranded (ds)DNA template molecule;
   (b) contacting the linear dsDNA template molecule with a first hairpin adaptor and a second hairpin adaptor to generate a closed linear DNA template molecule, wherein the closed linear DNA template molecule comprises at least one endonuclease target sequence;
   (c) amplifying the closed linear DNA template molecule to generate a double-stranded DNA molecule, wherein the closed linear DNA template molecule is amplified by rolling circle amplification in the presence of at least one type of a nuclease-resistant nucleotide; and
   (d) contacting the double-stranded DNA molecule with at least one endonuclease to generate the linear double-stranded DNA product, wherein the linear double-stranded DNA product has increased resistance to nucleases, such as exonucleases.
5. The method for any one of clauses 1-4, wherein the linear dsDNA template molecule comprises at least one endonuclease target sequence, and wherein step (b) further comprises contacting the linear dsDNA template molecule, the first hairpin adaptor and the second hairpin adaptor with an endonuclease, and optionally a ligase.
6. The method of any one of clauses 1-5, wherein the endonuclease target sequence of the linear dsDNA template molecule is different to the endonuclease target sequence of the closed linear DNA template molecule.
7. The method of any one of clauses 1-3 and 4-5, wherein the first hairpin adaptor and the second hairpin adaptor are different from the third and fourth adaptor molecules.
8. The method of any one of clauses 1-7, wherein the first hairpin adaptor and the second hairpin adaptor comprise a non-complementary loop region of at least 10 nucleotides, preferably at least 15 nucleotides.
9. The method of any one of clauses 1-8, wherein the at least one endonuclease in step (d), and step (b) (if present) is a Type IIS restriction endonuclease.
10. The method of any one of clauses 1-9, wherein the first and/or the second hairpin adaptor comprises a primase/polymerase recognition sequence, optionally wherein the primase/polymerase recognition sequence is located in the non-complementary loop region of the first hairpin adaptor and/or the second hairpin adaptor.
11.The method of any one of clauses 1-10, wherein the first harpin adaptor, the second hairpin adaptor, and, if present, the third adaptor and/or the fourth adaptor comprise an overhang or a blunt end.
12. The method of any one of clauses 1-11, wherein the method further comprises a step of purification of the linear DNA product after step (d), or, if present, step (e).
13. The method of any one of clauses 1-12, wherein the method further comprises a step of re-amplification of the DNA product.
14. The method of any one of clauses 1-13, wherein the method further comprises a step of exonuclease digestion after step (b) and/or step (d), or step (e), if present.
15. The method of any one of clauses 1-14, wherein the first hairpin adaptor and/or the second hairpin adaptor comprise an endonuclease targeting sequence.

### BRIEF DESCRIPTION OF THE DRAWINGS

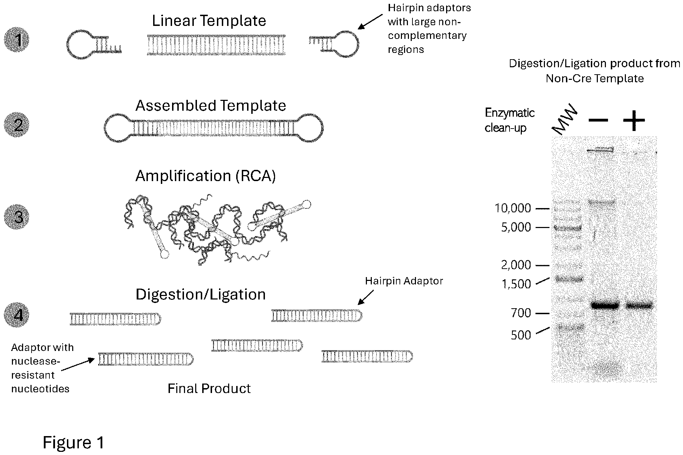

Figure 1 illustrates one of the methods of the present invention based on a non-Cre template strategy. In step 1, a linear dsDNA template molecule is contacted with a first hairpin adaptor and a second hairpin adaptor to form a closed linear DNA template molecule (step 2). In this example, both hairpin adaptors comprise overhangs. In step 3, the closed linear DNA template molecule is amplified by rolling circle amplification to form a double-stranded DNA molecule. In step 4, the double-stranded DNA molecule is contacted with third and fourth adaptor molecules (in a single contiguous aqueous volume) in the presence of an endonuclease and a ligase to generate a linear DNA product. In the example of Figure 1, the third adaptor comprises exonuclease-resistant nucleotides and the fourth adaptor is a hairpin adaptor; thus, the linear DNA product is a partially closed DNA product. The DNA product after digestion/ligation (DL) is optionally exposed to exonuclease digestion to digest any material (e.g. adaptors of partially ligated DNA molecules) that is not resistant to exonuclease digestion. The DNA product after exonuclease digestion is shown on an agarose gel (right side panel).

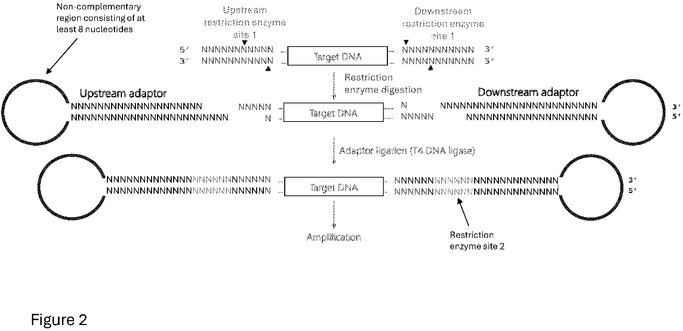

Figure 2 illustrates the endonuclease digestion and first and second hairpin adaptor ligation to a linear dsDNA template molecule to generate a closed linear DNA template molecule (i.e. steps (a) and (b) of the methods of the invention). The linear dsDNA template molecule comprises one type of the endonuclease target sequence, whereas the closed linear DNA template molecule comprises a different type of the endonuclease target sequence.

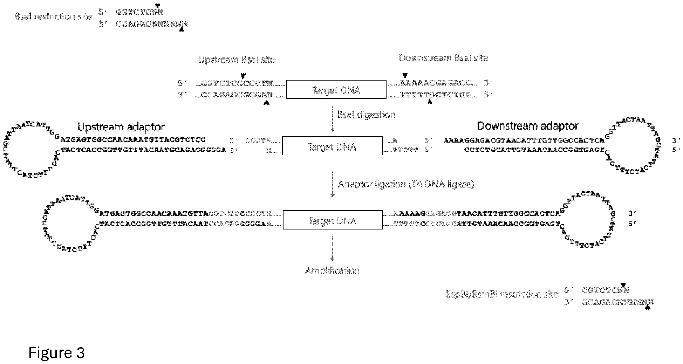

Figure 3 illustrates an example of the hairpin adaptors used in step (b) of the methods of the invention. Figure 3 shows the Bsal endonuclease digestion and first and second hairpin adaptor ligation to a linear dsDNA template molecule to generate a closed linear DNA template molecule (i.e. steps (a) and (b) of the methods of the invention). The linear dsDNA template molecule comprises one type of the endonuclease target sequence (here: a Bsal target sequences, which is located both 5' and 3' of the cassette (i.e. target DNA). Thus, the linear dsDNA template molecule comprises two endonuclease target sequences in this example. Both endonuclease target sequences are Bsal target sequences. The first (i.e. upstream) hairpin adaptor has a sequence of SEQ ID NO: 2 (in the non-self-hybridized form). The second (i.e. downstream) hairpin adaptor has a sequence of SEQ ID NO: 10 (in the non-self-hybridized form). Upon digestion with Bsal and ligation with T4 DNA ligase, the first hairpin adaptor and the second hairpin adaptor are appended to the linear dsDNA template molecule to form a closed linear DNA template molecule. The closed linear DNA template molecule comprises a different type of the endonuclease target sequence. In this example, the closed linear DNA template molecule comprises a Esp3I/BsmBI target sequence on each side (i.e. both 3' and 5') of the cassette (i.e. target DNA). The Esp3I/BsmBI target sequences are introduced into the closed linear DNA template molecule from the complementary regions of the first hairpin adaptor and the second hairpin adaptor.

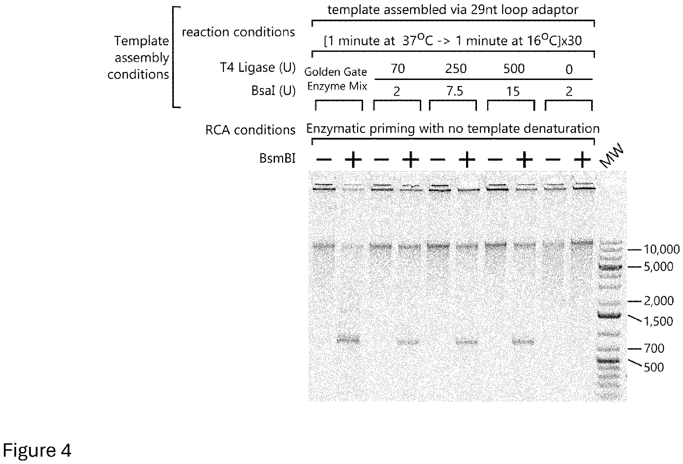

Figure 4 illustrates the DNA products of 796 base pairs produced by BsmBl digestion of RCA amplified material utilising the closed linear dsDNA templates with varying enzyme amounts. The presence of the 796bp DNA band in all digested lanes indicates the successful formation of the closed linear dsDNA template molecule during assembly, therefore the introduction of the Esp3I/BsmBI recognition site by the first hairpin adaptor and the second hairpin adaptor. No significant difference in the DNA product band intensity was observed between reactions containing different T4 ligase and Bsal-HFv2 unit quantities.

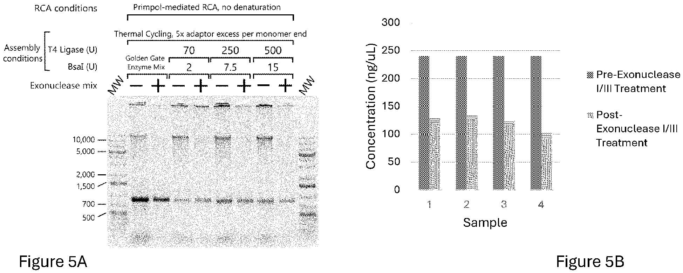

Figure 5A illustrates a -800 base pairs DNA product produced from the RCA reaction (and after digestion with an endonuclease and ligation of the third and fourth adaptor molecules). The maintained DNA band intensity post-exonuclease I and exonuclease III treatment (i.e. "exonuclease mix") of all samples indicates that the DNA product has been successfully appended to the third and fourth adaptor molecules. Protection from the exonucleolytic degradation is facilitated by the non-complementary loop region of the third adaptor molecule (which is a hairpin adaptor) and the presence of the phosphorothioate nucleotides in the fourth adaptor molecule. The exemplary sequence of the third adaptor molecule is:
   5'- [Phos] AGGGCTAACATTTGTTGGCCACTCAGGCCAACAAATGTTAG (SEQ ID NO: 7).
   The exemplary sequences of the fourth adaptor molecules are:
      5'-[Phos] AAAACTAACATTTGT*T*G*G*C*C (SEQ ID NO: 8) (first (e.g. top) strand); and
      5'-G*G*C*C*A*ACAAATGTTAG (SEQ ID NO: 9) (second (e.g. bottom) strand).
   In the sequences, the asterisk (*) denotes the presence of a phosphorothioate bond between two nucleotides.
Figure 5B shows a digestion/ligation reaction concentration pre and post exonuclease I/III treatment for each sample. All samples retaining roughly 50% of DNA post-exonuclease I/III treatment, which indicates adaptor ligation efficiency of -50%.
Figure 6 A illustrates examples of first and second hairpin adaptors which can be used in the methods of the invention.
Figure 6B (top) illustrates the closed linear DNA template molecule generated by the methods of the invention. Figure 6B (bottom) illustrates exemplary priming regions in the closed linear DNA template molecule for DNA primase/polymerase enzymes (e.g. TthPrimPol), which can be used in the methods of the invention (e.g. during rolling circle amplification).

### SEQUENCES DISCLOSED IN THE APPLICATION

**Table 1. Sequences disclosed in the application. "N" denotes any nucleotide. "*" denotes a phosphorothioated bond.**

| **SE Q ID NO** | **Sequence (5'-3')** | **Name** |
|---|---|---|
| 1 | AGGGGGAGACGTAACATTTGTTGGCCACTCATGGGAGGGTGTGT GGTCTTGGTTGGTGTGAGTGGCCAACAAATGTTACGTCTCC | Example of the hairpin adaptor |
| 2 | AGGGGGAGACGTAACATTTGTTGGCCACTCATCACTTTCTACTTA TCGATTAATCATTGGATGTGAGTGGCCAACAAATGTTACGTCTCC | Example of the hairpin adaptor |
| 3 | AGGGGGAGACGTAACATTTGTTGGCCACTCATATCCACTTTTCAC CATGTGAGTGGCCAACAAATGTTACGTCTCC | Example of the hairpin adaptor |
| 4 | GGGAGGGTGTGTGGTCTTGGTTGG | Example of the non-complementar y loop region |
| 5 | CACTTTCTACTTATCGATTAATCATTGGA | Example of the non-complementar y loop region |
| 6 | ATCCACTTTTCACCA | Example of the non-complementar y loop region |
| 7 | AGGGCTAACATTTGTTGGCCACTCAGGCCAACAAATGTTAG | Exemplary adaptor molecule |
| 8 | AAAACTAACATTTGT*T*G*G*C*C | One (e.g. top) strand of exemplary adaptor molecule |
| 9 | G*G*C*C*A*ACAAATGTTAG | One (e.g. bottom) strand of exemplary adaptor molecule |
| 10 | AAAAGGAGACGTAACATTTGTTGGCCACTCATCACTTTCTAC TTATCGATTAATCATTGGATGTGAGTGGCCAACAAATGTTACGTCTCC | Exemplary adaptor molecule |
| 11 | NAGGGGGAGACGTAACATTTGTTGGCCACTCATCACTTTCTAC TTATCGATTAATCATTGGATGAGTGGCCAACAAATGTTACGTCTCCCC CTN | Sequence from Figure 3 |
| 12 | AAAAAGGAGACGTAACATTTGTTGGCCACTCAGGTTACTAAT TAGCTATTCATCTTTCACTGAGTGGCCAACAAATGTTACGTCTCCTTTT T | Sequence from Figure 3 |

### EXAMPLES

### Example 1

An example of the method of the invention is shown in Figure 1. Figure 1 illustrates one of the methods of the present invention based on a non-Cre template strategy to generate a partially closed DNA product. In step 1, a linear dsDNA template molecule is contacted with a first hairpin adaptor and a second hairpin adaptor to form a closed linear DNA template molecule (step 2). In this example, both hairpin adaptors comprise overhangs. In step 3, the closed linear DNA template molecule is amplified by rolling circle amplification to form a double-stranded DNA molecule. In step 4, the double-stranded DNA molecule is contacted with third and fourth adaptor molecules (in a single contiguous aqueous volume) in the presence of an endonuclease and a ligase to generate a linear DNA product. In the example of Figure 1, the fourth adaptor comprises exonuclease-resistant nucleotides and the third adaptor is a hairpin adaptor (this can be reversed; i.e. the fourth adaptor molecule may be a hairpin adaptor, and the third adaptor molecule may comprise exonuclease-resistant nucleotides. Similarly, both adaptor molecules may be hairpins or both adaptor molecules may be linear adaptor molecules comprising exonuclease-resistant nucleotides); thus, the linear DNA product is a partially closed DNA product. The DNA product after digestion/ligation (DL) is optionally exposed to exonuclease digestion to digest any material (e.g. adaptors of partially ligated DNA molecules) that is not resistant to exonuclease digestion. The DNA product after exonuclease digestion is shown on an agarose gel (right side panel).

Figure 3 illustrates an example of the hairpin adaptors used in step (b) of the methods of the invention. Figure 3 shows the Bsal endonuclease digestion and first and second hairpin adaptor ligation to a linear dsDNA template molecule to generate a closed linear DNA template molecule (i.e. steps (a) and (b) of the methods of the invention). The linear dsDNA template molecule comprises one type of the endonuclease target sequence (here: a Bsal target sequences, which is located both 5' and 3' of the cassette (i.e. target DNA). Thus, the linear dsDNA template molecule comprises two endonuclease target sequences in this example. Both endonuclease target sequences are Bsal target sequences. The first (i.e. upstream) hairpin adaptor has a sequence of SEQ ID NO: 2 (in the non-self-hybridized form). The second (i.e. downstream) hairpin adaptor has a sequence of SEQ ID NO: 10 (in the non-self-hybridized form). Upon digestion with Bsal and ligation with T4 DNA ligase, the first hairpin adaptor and the second hairpin adaptor are appended to the linear dsDNA template molecule to form a closed linear DNA template molecule. The closed linear DNA template molecule comprises a different type of the endonuclease target sequence. In this example, the closed linear DNA template molecule comprises a Esp3I/BsmBI target sequence on each side (i.e. both 3' and 5') of the cassette (i.e. target DNA). The Esp3I/BsmBI target sequences are introduced into the closed linear DNA template molecule from the complementary regions of the first hairpin adaptor and the second hairpin adaptor.

Prior to contacting the first hairpin adaptor and the second hairpin adaptor with the linear dsDNA template molecule, the adaptors are self-hybridized separately at 100µM concentration in 1x hybridization buffer (20 mM Tris HCl pH 7.5, 300 mM NaCl) by incubating adaptors at 80°C for 10 minutes, then gradually cooling to a room temperature (about 22°C) overnight.

Self-hybridized adaptors (i.e. the first and second hairpin adaptors) in this example comprise a 29 nucleotides long non-complementary loop region. The first and second hairpin adaptors each comprise a 4-nucleotide long overhangs (here: 5' GGGA and AAAA). The first and second hairpin adaptors each comprise a primase/polymerase recognition sequence (i.e. XTC) in the non-complementary loop region.

Experimental conditions for the steps shown in Figures 3 and 4: 75ng of the PCR amplified DNA fragment (i.e. the linear dsDNA template molecule) was used per reaction in a final reaction volume of 20µL: 2µL of the 10X T4 ligase buffer (4basebio), 1 mM ATP (4basebio), 0.72ng/µL of the first hairpin adaptor (SEQ ID NO: 2 in the non-self-hybridized form), 0.72ng/µL of the second hairpin adaptor (SEQ ID NO: 10 in the non-self-hybridized form) and, varying units of Bsal-HFv2 (New England Biolabs) and T4 ligase (New England Biolabs) in each reaction (see Figure 4 for details). Alternatively, a singular reaction containing 2µL of NEBridge^{®} Golden Gate Enzyme Mix (Bsal-HFv2) (New England Biolabs) (termed "Golden Gate Enzyme Mix" in Figure 4) instead of the separate enzyme components was used to test this kit. The reactions were incubated in a single reaction pot for [1 minute at 37°C then 1 minute at 16°C] for 30 cycles followed by 5 minutes at 60°C using the C1000 Touch Thermal Cycler (Biorad).

After digestion and ligation, 0.08ng/uL of Exonuclease III (4basebio) was added and each reaction was incubated for 1 hour at 37°C then 10 minutes at 65°C. Post-exonuclease III treatment, sample concentrations were measured using Qubit quantification with 1µL sample load via Qubit DNA High Sensitivity Kit (Invitrogen) and the results are visualized in Figure 4.

Shown in Figure 4 are the DNA products of 796 base pairs produced by BsmBl digestion of RCA amplified material utilising the closed linear dsDNA template molecules with varying enzyme amounts. The presence of the 796bp DNA band in all digested lanes indicates the successful formation of the closed linear dsDNA template molecule during assembly, therefore the introduction of the Esp3I/BsmBI recognition site by the first hairpin adaptor and the second hairpin adaptor. No significant difference in the DNA product band intensity was observed between reactions containing different T4 ligase and Bsal-HFv2 unit quantities. However, a notable increase in the DNA product generated using the NEBridge^{®} Golden Gate Enzyme Mix is apparent, and the amount of undigested RCA material is reduced. The NEBridge^{®} Golden Gate Enzyme Mix is likely to have a different ratio of enzymes (as compared to the enzymes used without the kit, as discussed above). The control RCA reaction (absence of T4 ligase in the assembly reaction) shows no digestion with Bsmbl.

The RCA reaction conditions: 15 ng of the closed linear dsDNA template molecule was used per sample in a final reaction volume of 150µL; 15µL of the 10x RCA Buffer (4basebio), 1 mM of the dNTPs (Promega), 20mM of KCI (Sigma-Aldrich), 0.2ng/uL of Pyrophosphatase (4basebio), 3.4 ng/µL of PrimPol DNA primase/polymerase (4basebio), 16ng/µL of QualiPhi DNA polymerase (i.e. a chimeric form of Phi29 DNA Polymerase) (4basebio). The reaction was incubated for 20 hours at 30°C, followed by 10 minutes at 55°C.

BsmBl digestion reaction conditions: 10µg of each RCA-produced material was digested in a 100µL final reaction volume: 10µL of the 10x CutSmart Buffer (New England Biolabs) and 4U/µg of DNA BsmBlv2 (New England Biolabs). The reaction was incubated for 1 hour at 37°C. After 1 hour, another 4U/µg of DNA BsmBlv2 (New England Biolabs) was added and each reaction was incubated for a further 2 hours at 37°C.

Figure 5A illustrates a -800 base pairs DNA product produced from the RCA reaction (and after digestion with BsmBl). The maintained DNA band intensity post-exonuclease I and exonuclease III treatment (i.e. "exonuclease mix") of all samples indicates that the DNA product has been successfully appended to the third and fourth adaptor molecules. Protection from the exonucleolytic degradation is facilitated by the non-complementary loop region of the third adaptor molecule (which is a hairpin adaptor) and the presence of the phosphorothioate nucleotides in the fourth adaptor molecule. The exemplary sequences of the third adaptor molecule and the fourth adaptor molecule are SEQ ID NO: 7 (e.g. third adaptor), and SEQ ID NO: 8 and SEQ ID NO: 9 (e.g. two complementary strands of the fourth adaptor), respectively.

Displayed in Figure 5B is a digestion/ligation reaction concentration pre- and post- exonuclease I/III treatment (i.e. "exonuclease mix") for each sample as measure by Qubit BR assay (ThermoFisher). All samples retained approximately 50% of DNA post-exonuclease I/III treatment, which indicates adaptor ligation efficiency of -50%.

### Exonuclease digestion reaction conditions:

The enzymatic purification was performed by supplying12.9ng/µL of Exonuclease I (4basebio), and 6.225ng/µL of Exonuclease III (4basebio), which were added directly into the reaction and incubated for 2 hours at 37°C.

## Claims

1. A method for producing a closed linear deoxyribonucleic acid (DNA) product, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the third adaptor molecule and closed at a second end by the fourth adaptor molecule.

2. A method for producing a linear deoxyribonucleic acid (DNA) product, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the linear DNA product, wherein the linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third and fourth adaptor molecules are nucleic acid molecules that comprise one or more nuclease-resistant nucleotides.

3. A method for producing a partially closed deoxyribonucleic acid (DNA) product, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification;
(d) contacting the double-stranded DNA molecule with an endonuclease, a ligase and third and fourth adaptor molecules to form a single contiguous aqueous volume; and
(e) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the third adaptor molecule is ligated to a first end of the linear double-stranded region and the fourth adaptor molecule is ligated to a second end of the linear double-stranded region, and wherein the third adaptor molecule is a nucleic acid molecule that comprise one or more nuclease-resistant nucleotides, and wherein the linear double-stranded region is closed at the second end by the fourth adaptor molecule.

4. A method for producing a linear double-stranded DNA product, wherein the method comprises:
(a) providing a linear double stranded (ds)DNA template molecule;
(b) contacting the linear dsDNA template molecule with a first hairpin adaptor and a second hairpin adaptor to generate a closed linear DNA template molecule, wherein the closed linear DNA template molecule comprises at least one endonuclease target sequence;
(c) amplifying the closed linear DNA template molecule to generate a double-stranded DNA molecule, wherein the closed linear DNA template molecule is amplified by rolling circle amplification in the presence of at least one type of a nuclease-resistant nucleotide; and
(d) contacting the double-stranded DNA molecule with at least one endonuclease to generate the linear double-stranded DNA product, wherein the linear double-stranded DNA product has increased resistance to nucleases, such as exonucleases.

5. The method for any one of claims 1-4, wherein the linear dsDNA template molecule comprises at least one endonuclease target sequence, and wherein step (b) further comprises contacting the linear dsDNA template molecule, the first hairpin adaptor and the second hairpin adaptor with an endonuclease, and optionally a ligase.

6. The method of any one of claims 1-5, wherein the endonuclease target sequence of the linear dsDNA template molecule is different to the endonuclease target sequence of the closed linear DNA template molecule.

7. The method of any one of claims 1-3 and 4-5, wherein the first hairpin adaptor and the second hairpin adaptor are different from the third and fourth adaptor molecules.

8. The method of any one of claims 1-7, wherein the first hairpin adaptor and the second hairpin adaptor comprise a non-complementary loop region of at least 10 nucleotides, preferably at least 15 nucleotides.

9. The method of any one of claims 1-8, wherein the at least one endonuclease in step (d), and step (b) (if present) is a Type IIS restriction endonuclease.

10. The method of any one of claims 1-9, wherein the first and/or the second hairpin adaptor comprises a primase/polymerase recognition sequence, optionally wherein the primase/polymerase recognition sequence is located in the non-complementary loop region of the first hairpin adaptor and/or the second hairpin adaptor.

11. The method of any one of claims 1-10, wherein the first harpin adaptor, the second hairpin adaptor, and, if present, the third adaptor and/or the fourth adaptor comprise an overhang or a blunt end.

12. The method of any one of claims 1-11, wherein the method further comprises a step of purification of the linear DNA product after step (d), or, if present, step (e).

13. The method of any one of claims 1-12, wherein the method further comprises a step of re-amplification of the DNA product.

14. The method of any one of claims 1-13, wherein the method further comprises a step of exonuclease digestion after step (b) and/or step (d), or step (e), if present.

15. The method of any one of claims 1-14, wherein the first hairpin adaptor and/or the second hairpin adaptor comprise an endonuclease targeting sequence.
